# EUROPEAN PATENT APPLICATION

(11) **EP 2 377 565 A1**
(43) Date of publication of application: **19.10.2011**
(21) Application number: 11005267.7
(22) Date of filing: 27.09.2004
(51) Int. Cl.: A61M 5/34, A61M 39/10

(54) **Medical syringe**

(30) Priority: 25.09.2003 JP 2003334136; 25.09.2003 JP 2003334135
(62) Divisional of application: 04788213.9
(71) Applicant: JMS Co., Ltd., Hiroshima-shi, Hiroshima 7308652 (JP)
(72) Inventor: Hongo, Susumu, Izumo-shi Shimane 693-0027 (JP); Ishi, Shigeaki, Hiroshima 731-2104 (JP); Kiyono, Takafumi, Hiroshima-shi Hiroshima 731-0221 (JP); Kondo, Ken, Hiroshima-shi Hiroshima 733-0007 (JP); Kunishige, Takahiko, Hiroshima-shi Hiroshima 731-0137 (JP); Morimoto, Masayuki, Akitakata-shi Hiroshima 731-0501 (JP); Okiyama, Tadashi, Hiroshima-shi Hiroshima 733-0815 (JP); Uematsu, Raita, Hiashihiroshima-shi Hiroshima 739-2115 (JP); Yuki, Takehiko, Hiroshima-shi Hiroshima 730-0847 (JP)
(74) Representative: Grünecker, Kinkeldey, Stockmair & Schwanhäusser

(57) **Abstract**

A medical syringe (1100), comprising a tubular connection assist member (1130) increasing the holding force of a luer part (1140) inserted into a connected part (1200). The connection assist member is formed to be slid on the luer part or a body part in the state of being movable between a first position near the tip of the luer part and a second position apart from the tip of the luer part. When the connection assist member is moved to the second position, the tip of the luer part is exposed.

## Description

### Technical Field

The present invention relates to a medical use syringe, and in particular to technology for enhancing syringe versatility.

### Background Art

Related prior art is disclosed in EP 0 716 860, US 5,047,021, GB 2 216 405, US 4,266,815 and EP0204311.

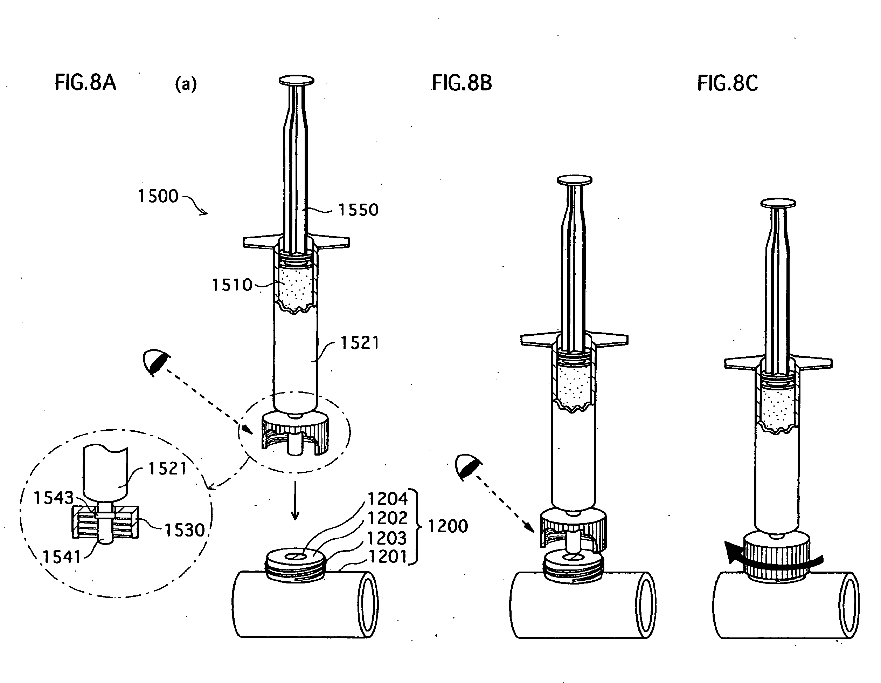

In recent years, a wide variety of types of medical syringe, including so-called injection tubes and the like, have been manufactured. A syringe having a lock mechanism for securely connecting to a specified port is one of those that have been proposed.
More specifically, a syringe 1500, as shown in FIG 8A, includes a tubular lure 1541 extending from one end of a syringe body 1521 for holding a liquid medication 1510 and having a stepped part 1543 provided towards the center thereof, a cylindrical locknut 1530 inserted between the stepped part 1543 and an end of the syringe body 1521, and a plunger 1550 inserted into an opening in the syringe body 1521.

A lure lock coinfusion port 1200 consists of a rubber valve 1204 that forms an insertion point of the lure 1541 of the syringe 1500, held in place by a cylindrical cover body 1202 on a side of a tube body 1201 that forms a transfusion line or similar flow pass.
Further, a thread groove 1203 for screwing with the locknut 1530 is provided on an external periphery surface of the cover body 1202.

A user who wants to connect the syringe 1500 to the lure lock coinfusion port 1200 has to tighten the locknut 1530 while blindly inserting the lure 1541 into a slit in the valve 1204.
Note that syringes having a lock mechanism that uses a connection supporting implement such as the locknut 1530 to prevent the syringe from easily separating from a confusion port or similar connection target are known as "lure lock syringes".

Contrastingly, syringes which permit simple insertion and removal in relation to a connection target implement are known as "lure slip syringes".
More specifically, lure slip syringes differ from lure lock syringes in that they do not include a connection supporting implement such as the locknut 1530.
Further, a lure slip coinfusion port 1210 differs from the lure lock coinfusion port 1200 in that it is not provided with a fitting part such as the thread groove 1203 that corresponds to the locknut 1530.

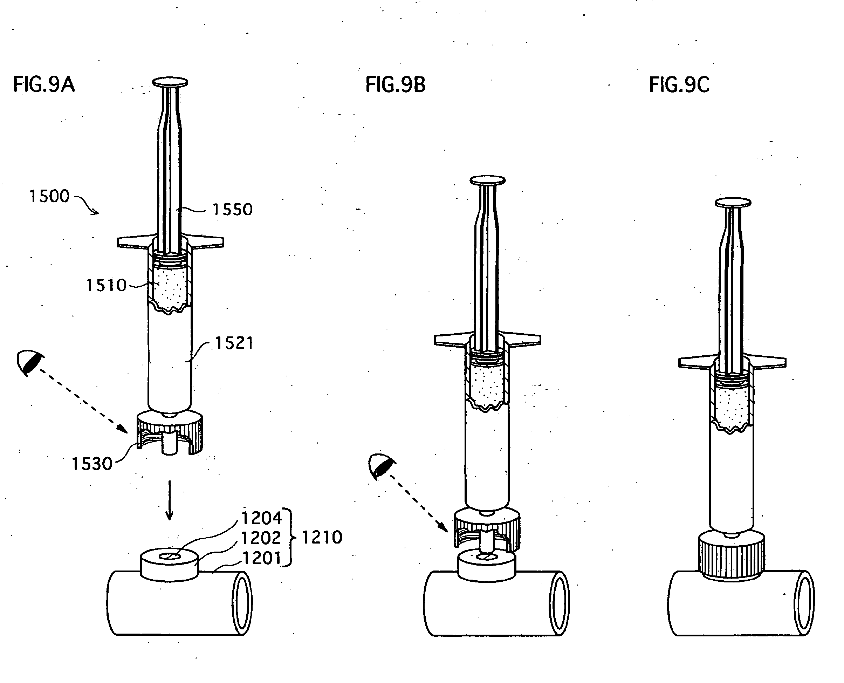

Normally, a lure lock syringe is used in combination with a lure lock coinfusion port, or a lure slip syringe with a lure slip coinfusion port.
A syringe having the connection supporting implement has the following problem. As shown in FIGs. 9A and 9B, the locknut 1530 hides the lure 1541, and consequently, a user attempting to connect the syringe 1500 to a lure slip coinfusion port 1210 is unable, given a normal working stance, to the see the tip of the lure 1541 during insertion into a slit in the valve 1204. The user is therefore unable to insert the syringe smoothly and needs time to complete the task.

In the case of the lure slip coinfusion port 1210, speedy insertion and withdrawal of the syringe are assumed, and it is not desirable if time is needed for the insertion.
A further problem is that both types of coinfusion port are in use in the medical field, and it is therefore conceivable that a lure lock syringe will be used in combination with the lure slip coinfusion port 1210, as shown in FIGs. 9A and 9B.

When the lure lock syringe is used in combination with the lure slip coinfusion port 1210, the syringe functions as a slip type syringe whereby the syringe is easily disengaged from the lure slip coinfusion port 1210. However, since it was not originally imagined that the lure lock syringe would be used in this way, the cover body 1202 of the lure slip coinfusion port 1210 may interfere with the locknut 1530 of the syringe 1500 when attempting to insert the syringe 1500, preventing connection.

Thus, immediate improvement is desired in the versatility of syringes that include a connection supporting member due to the requirements for speedy and accurate responses in the medical field.
There is currently a fundamental problem in the medical field due to the coexistence of both the lure slip and lure lock connection methods as forms of connection between the lure and connection targets such as coinfusion ports, needle hubs, and the ends of catheters.

Specifically, with transfusion circuits, blood circuits and the like, connection targets such as catheters (tubes) and coinfusion ports are generally integrated. Thus, if the style of connection used in the syringe is fixed and differs from that of one or more of the connection targets, circumstances may arise in which the connection cannot be made.
Such circumstances are undesirable in the medical field where speedy treatment is desired, requiring an operator to make extra effort and pay extra attention during preparation to examine whether the forms of connection match.

Although, on account of safety considerations and the like, there have recently been efforts to force a general introduction of the lure lock of arrangement which provides a very secure connection, this has not been legislated for, and a continuation of the present state of coexistence is predicted.

### Disclosure of the Invention

An object of the present invention, which was conceived in the light of the above problem, is to provide a highly versatile syringe that can be connected to a cylindrical a connection target without problems arising, whether or not the connection target has a locking mechanism. Here "the connection target" is an object into which the lure of the syringe is inserted to make a connection, and is generally a port, the end of a catheter, a needle hub, or the like.

In order to achieve the above object, the medical syringe of the present invention has a syringe unit including a lure and a syringe body; and a cylindrical connection supporting member that is slidably or detachably provided on the syringe unit, and increases a holding force of the lure when the syringe unit is in a connected state.
Whether it is a lure slip style or lure lock style that is selected, this construction enables lure connection in the selected style by the connection supporting member being either slid to a prescribed position, or being attached/detached. Consequently, the awkwardness (delays in treatment, complicated preparation etc.) that occurred due to the inflexible nature of the connection means in the conventional syringe does not occur, and treatments can be administered quickly.

In the slidable construction, the connection supporting member is formed to be slid along the lure or the syringe body in a state of being movable between a first position near a tip of the lure and a second position away from the tip of the lure, while remaining engaged with the syringe unit.
Describing the construction of the syringe in more detail, in order achieve the above object, the medical syringe of the present invention includes: a syringe unit including a lure for inserting into a connection target, and a syringe body; and a connection supporting member that increases a holding force of the lure when the syringe unit is connected to the connection target, wherein the connection supporting member is slidably provided on the lure or the syringe body in a state of being movable between a first position near a tip of the lure and a second position away from the tip of the lure, and when the connection supporting member is moved to the second position, the tip of the lure is exposed.

Normally, the connection supporting member engages with the connection target near the tip of the lure, and so when the above construction is to be usedas a lure lock syringe, moving the connection supporting member to the first position enables the connection, supporting member to engage with the connection target to increase the holding force. Moreover, when the lure is inserted into the connection target, moving the connection supporting member to the second position enables a quick connection by improving the visibility of the location where the connection is being performed.

When using the above construction as a lure slip syringe, since interference between the connection supporting member and the connection target (port) can be avoided, the medical syringe can be used without inconvenience, and its versatility increased.
It is preferable that the medical syringe further includes: a contact part that is provided on the lure or on the syringe body and contacts the connection supporting member when the connection supporting member is moved to the first position, to prevent movement of the connection supporting member from the first position towards the tip of the lure.

According to the above construction, with the lure inserted into the target object, the relative positions of the syringe and the connection target are fixed by engaging the connection supporting member with the connection target.
It is also preferable that the sliding of the connection supporting member is realized by inserting the lure or syringe body into a through hole provided in the connection member, the contact part is a large diameter part provided on an external periphery of the lure or the syringe body, and spiral grooves are provided in a peripheral surface of the contact part.

According to the above construction, the fitting of the connection supporting member over the contact part can be realized by rotating the connection supporting member onto the spiral grooves. Thus, the load on the person assembling the syringe can be reduced.
It is further preferable that, when in the second position, the connection supporting member is fitted to the lure or the syringe body by a holding force that is at least sufficient to prevent the connection supporting member dropping under own weight.
According to the above construction, the positional relation of the connection supporting member and the lure or syringe body is fixed when the connection supporting member is moved to the second position. Thus, the connection supporting member will not change its position by dropping due to its own weight.

The present invention is also a medical syringe including a lock connector for connecting the syringe to a connection target, wherein the lock connector includes a cylindrical connector body, a syringe body is inserted through the connector body and an elastic body is interposed between an external surface of the syringe body and an internal surface of the connector body, and the syringe body is held with the connector body by a restoring force of the elastic body.

The elastic body may be composed of a spring body or an elastomer.
In these constructions, the elastic body contacts the syringe surface, and the syringe body is therefore held against the connector body by the restoring force of the elastic body. Thus, by connecting the connector body to the connection target it is also possible to fix the syringe body to the connection target.
Further, an operator applying an external force to the connector body in the syringe axial direction enables the lock connector to slide over the surface of the syringe body, with the following effect.

Namely, if the sliding operation is performed, for instance, to slide the connector body to the posterior end of the syringe body, the lure region at the tip of the syringe body can be exposed while the syringe body is held against the connector body by the restoring force of the elastic body. Thus, since the lure region is freely exposed and the position of the tip can be easily checked by eye, operations like connection to a direction connection-type port, or attachment to a needle hub can be performed with ease.

On the other hand, if the sliding operation is performed, for instance, to slide the connector body to the lure end of the syringe body, the external surface of the syringe body near the lure is hidden inside the connector body while the connector body is held to the syringe body by a frictional force. In this state, the lock connector can be screwed onto the fixed connection-type connection target (the fixed connection-type port) using a thread formed in the lock connector, and the lure of the syringe favorably connected to the fixed connection-type connection target.

In order to achieve the above object, the present invention is further a medical syringe including a lock connector for connecting the syringe to a connection target, wherein the lock connector has a cylindrical connector body and a protrusion formed on an internal surface of the connector body, a syringe body has a return groove that extends in a syringe axial direction provided in an external surface thereof, and the syringe body has an engagement groove that communicates with the return groove and extends in a syringe circumferential direction. The lock connector can be held so as to be moveable back and forth, the syringe body being inserted into the connector body, and the protrusion of the connector body being fitted in the return groove of the syringe body. The syringe body and the connector body can also be engaged by the protrusion being fitted into the engagement groove.

Here, the engagement groove is provided towards the tip of the syringe body so as to communicate with the return groove.
In the medical syringe including the lock connector, the connector body is movable back and forth on the syringe body by the operator operating on the connector body to slide the protrusion in the return groove that extends in the syringe axial direction.

Thus, if the connector body is slid to the posterior end of the syringe body, the lure region at the tip end of the syringe body can be exposed, and operations such as connection to a direct connection-type connection target (port), or attachment to a needle hub can be performed with ease.

Further, if the operator moves the lock connector to fit the protrusion of the lock connector into the return groove and then fit the protrusion into the engagement groove that communicates with the return groove, the lock connector is fixed in the axial direction while being held by the syringe body. Consequently, lock connector movement can be restricted to the location where the engagement groove is formed, and it is possible to reduce play and securely fix the lock connector in position.

Note that if this kind of engagement groove is provided towards the tip of the syringe, the lock connector and the syringe body can be fixed in position with the lock connector covering the lure, enabling the syringe body to be connected to the fixed connection-type port without any play and stability over the period of connection to be ensured. The detachable construction of the present invention for achieving the above object may be realized by a construction in which the connector member is detached from the syringe by a prescribed disengagement operation. This prescribed disengagement operation is described in detail below.

In order to achieve the above object the medical syringe of the present invention has a syringe unit including a lure for inserting into a connection target, and a syringe body; and a connection supporting member that increases a holding force of the lure when the syringe unit is connected to the connection target, wherein one of the lure and the connection supporting member has a small diameter portion sandwiched between large diameter portions, the other of the lure and the connection supporting member has a first member that has a first opening in a main surface thereof, and a second member that has a second opening in a main surface thereof, the first and second members oppose one another with the main surfaces thereof near or contacting one another, and at least one of the members is displaceable relative to the other member, such that a hole in plan view formed by the first and second openings overlapping changes between a first size that permits insertion of the large diameter portion and a second size that prevents insertion of the large diameter portion.

When attaching the connection supporting member to the syringe unit, the first and second members are displaced relative to each other to make the hole the first size, and the lure is inserted into the hole as far as the small diameter portion. Then, the first and second members are again displaced relative to each other to make the hole the second size.

Consequently, the connection supporting member can be simply detached from the lure of the medical use syringe.
Thus, with the connection supporting member attached to the lure or the medical use syringe, the medical use syringe of the present invention can be connected to a so-called lure lock connection target. On the other hand, with the connection supporting member detached, the medical use syringe of the present invention can be connected to a so-called lure slip connection target.

Moreover, the present invention has been arranged so that the screwing onto the lure-lock connection target can be executed quickly and easily when the lure lock syringe is chosen. This medical syringe has a syringe unit including a lure and a syringe body; and a cylindrical connection supporting member that is rotatably provided on the syringe unit, and increases the holding force of the lure when the syringe unit is in a connected state.
Furthermore, in this medical syringe, the connection supporting member is slidable or detachable to enable the conversion from a lure slip syringe to a lure lock syringe to be quickly and easily executed.

### Brief Description of the Drawings

FIG. 1A illustrates the state of the lure lock syringe of a first embodiment before insertion into the lure lock coinfusion port 1200, FIG. 1B illustrates the state of the lure lock syringe of the first embodiment after insertion into the lure lock coinfusion port 1200, FIG. 1C illustrates the state of the syringe of the first embodiment after locking;

FIG. 2A illustrates the state of the lure slip syringe of the first embodiment before insertion into the lure slip coinfusion port, FIG. 2B illustrates the state of the lure slip syringe of the first embodiment after insertion into the lure slip coinfusion port;
FIG. 3A shows a condition directly before passing a locknut over a protruding part,

FIG. 3B shows a condition when passing the locknut over the protruding part, FIG. 3C shows a condition after passing the locknut over the protruding part;
FIG. 4 shows an example of a locknut throughhole for facilitating the passing of the locknut over the protruding part;
FIG. 5A illustrates a syringe of a second embodiment before insertion into the lure lock coinfusion port,

FIG. 5B illustrates the syringe of the second embodiment after insertion into the lure lock coinfusion port, FIG 5C illustrates syringe of the second embodiment after locking;
FIG. 6A illustrates a syringe of the second embodiment before insertion into the lure slip coinfusion port,

FIG. 6B illustrates the syringe of the second embodiment after insertion into the lure slip coinfusion.port;
FIG. 7 shows an example of a syringe locking mechanism realized by means other than a nut;
FIG. 8A illustrates a conventional lure lock syringe before insertion into the lure lock coinfusion port,

FIG. 8B illustrates the conventional lure lock syringe after insertion into the lure lock coinfusion port, FIG 8C illustrates a conventional lure lock syringe after locking;
FIG. 9A illustrates the conventional lure lock syringe before insertion into the lure slip coinfusion port,

FIG. 9B illustrates the conventional lure lock syringe during insertion into the lure slip coinfusion port,
FIG. 9C illustrates the conventional lure lock syringe after insertion into the lure slip coinfusion port;

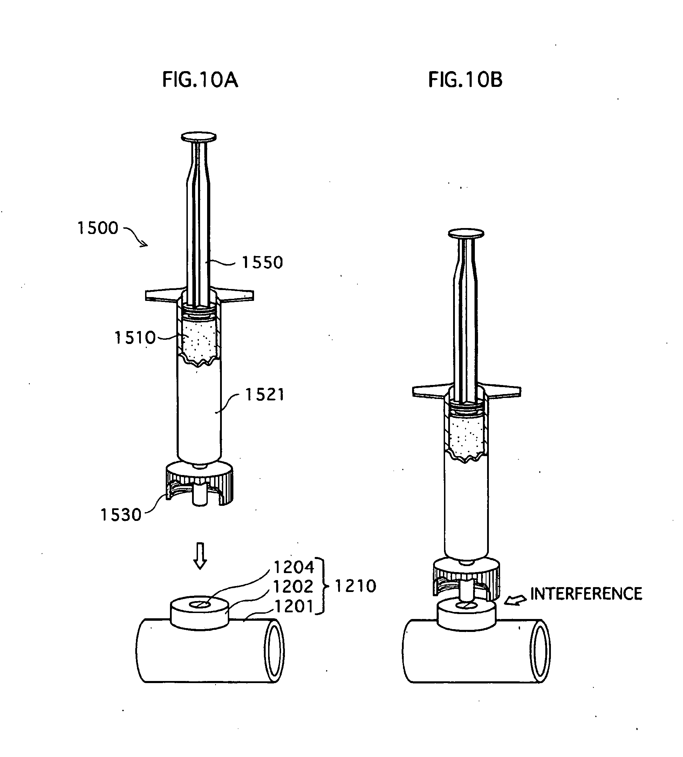

FIGs. 10A and 10B illustrate a problem that occurs when the conventional lure lock syringe is inserted into the lure slip coinfusion port;

FIG. 11A shows the syringe of a third embodiment before lock attachment, FIG. 11B shows the syringe of the third embodiment after lock attachment being connected to the lure lock coinfusion port;
FIGs. 12A, 12B, 12C, and 12D show the positional relation of a lock body and a slider plate during operation in the third embodiment;

FIG. 13 shows a lure slip syringe of the third embodiment after lock removal being connected to the lure slip coinfusion port;
FIG. 14 a modification of the slider plate of the lock in the third embodiment;
FIGs. 15A, 15B, 15C, and 15D show the positional relation of the lock body and the slider plate during in a fourth embodiment;

FIG. 16 shows a construction of a syringe with lock connector of a fifth embodiment;
FIG. 17 shows a construction of a syringe with attached lock connector when connected to a fixed connection-type port;
FIG. 18 shows a construction of a syringe with lock connector when connected to a direct connection-type port;

FIG. 19 shows a construction of a syringe with lock connector when connected to a needle hub;
FIG. 20 shows a construction of a syringe with lock connector of the fifth embodiment;
FIG. 21 shows a construction of a syringe with lock connector of a sixth embodiment when connected to the fixed connection-type port;
FIG. 22 shows a construction of the syringe with lock connector of the sixth embodiment when connected to the fixed connection-type port;

FIG. 23 shows a construction of a syringe with lock connector of a seventh embodiment when connected to the fixed connection-type port;
FIG. 24 is an exterior view of a syringe body of the seventh embodiment;
FIG. 25 shows a construction of the syringe with lock connector of the seventh embodiment when connected to the direct connection-type port;

FIGs. 26A and 26B show a construction of a conventional syringe; and
FIGs. 27A and 27B show a construction of a syringe of an eighth embodiment.

### Best Mode for Carrying Out the Invention

The present invention realizes both lure slip and lure lock connections by enabling a connector member that supports locked connection to slide relative to a syringe body or be removed from the syringe body. Examples of sliding methods include those realized hear a base of the lure, and those realized on the tubular body of the syringe. In both cases , excellent operability is achieved because the connector member and syringe remain engaged rather than being separated both before/after and during the sliding. So as to allow detachment, a construction in which the connecting member can be detached from the syringe by a predetermined detachment operation is proposed. With this construction, the connector member can be detached using a simple operation when not required, and simply and quickly attached when required. Moreover, the present invention provides a syringe characterized by a locking member that is rotatable when attached to the syringe. This type of mechanism already exists for glass syringes but is new and unique in the plastic syringes now mainly used in the medical field, and has practical use value. As the locking member is rotatable, it can be simply and quickly secured in a one-handed operation. Further, the present invention provides a syringe having guiding grooves (guide parts) so as to enable simpler and faster attachment of a locking member. Though it is necessary to attach the locking member when the lure lock arrangement is selected, this can be performed simply and quickly using the guiding grooves.

The following describes the first through eighth embodiments of the medical syringe of the present invention with reference to the drawings.

### First Embodiment

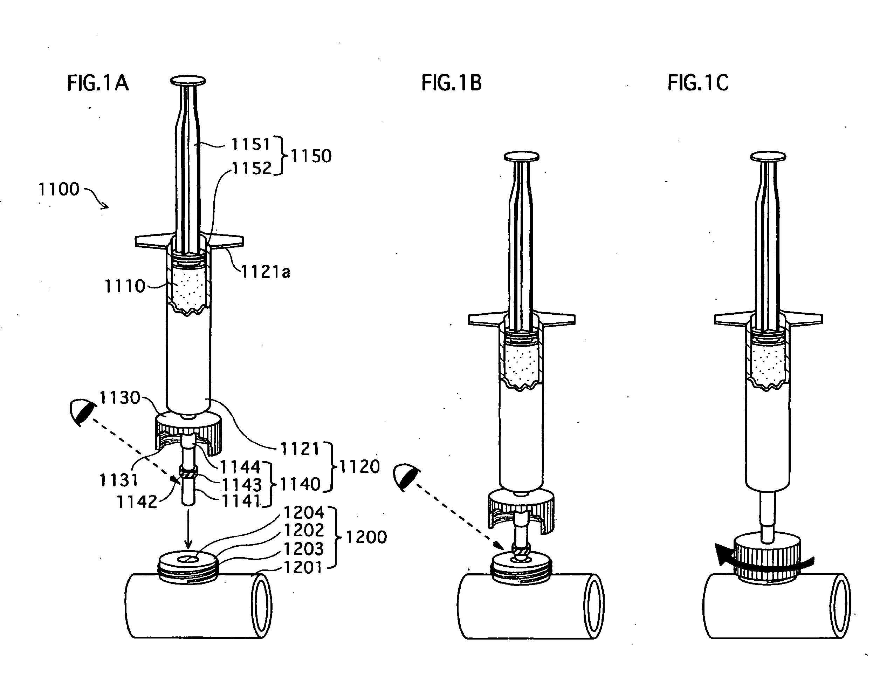

FIGs. 1A, 1B and 1C illustrate a syringe of the first embodiment.

The syringe 1100 is a pre-filled syringe which has been filled with a liquid medication 1110, and which can be quickly inserted and removed in relation to both lure lock coinfusion ports and lure slip coinfusion ports.
This syringe 1100 is formed by filling a tubular syringe part 1120 with the liquid medication 1110, and then sealing the syringe part 1120 with a plunger 1150, and installing a locknut 1130 at one end of the syringe part 1120.
The syringe part 1120 is formed from a tubular extending part 1140 extending from one end (hereinafter referred to as "the first end") of a tubular syringe body 1121, and a flange 1121 a provided at the opposite end to the first end (hereinafter referred to as "the second end").
The extending part 1140 is formed from a tubular first lure part 1144 connected to the first end, a tubular second lure part 1141 that extends from the end of the first lure part 1144 not connected to the first end, and a substantially cylindrical stopper 1143 peripherally provided in a central region of the second lure part 1141.

The stopper 1143 is tapered such that a diameter d1' of the stopper 1143 on the side where the tip of the second lure part 1141 is located is smaller than a diameter d1 on the side where the first lure part 1144 is located. Moreover, the stopper 1143 has spiral grooves provided in an external surface thereof.
Further, if the external diameter of the first lure part 1144 is d2, and the external diameter of the second lure part 1141 is d3, the relationship between them will be d1 > d2 > d3.

The locknut 1130 is a cylindrical nut that includes a base and is composed of a flexible resin material suitable engaging witch an implement such as an coinfusion port or the like, and a through hole of inner diameter d4 is provided in a central portion of the base.
The extending part 1140 is inserted through this through hole such that the locknut 1130 is movable between the first end and the stopper 1143.

The positional relation of the first lure part 1144, the second lure part 1141, and the stopper 1143 is set such that the tip end of the second lure part 1141 (and preferably the stopper 1143) is exposed when the locknut 1130 is positioned nearest the first end.
Here, the position at which the tip end of the second lure part 1141 and preferably the stopper 1143 are exposed refers to a position at which a person connecting the syringe 1100 and the lure lock coinfusion port 1200 or the lure slip coinfusion port 1210 in a normal working position is able to see the tip end of the second lure part 1141 and preferably the stopper 1143.

The value of d4 is set such that the locknut 1130 but can be moved easily by hand when the through hole is fitted to the first lure part 1144 and to the portion of the second lure part 1141 up from the stopper 1143. Here, the fit is tighter when the locknut 1130 is fitted to the first lure part 1144 than when it is fitted to the portion of the second lure part 1141 up from the stopper 1143.

In summary, the values of d2 and d3 are very close to the value of d4, but are a little larger than the value of d4. Moreover, d4 ≥ d1'.
The plunger 1150 has rubber packing 1152 peripherally provided at one end of a plunger body part 1151.
The lure lock coinfusion port 1200 consists of the rubber valve 1204 having a slit and held in place by the cylindrical cover body 1202 on the side of the tube body 1201 that forms a transfusion line or similar flow pass. The thread groove 1203 is provided on an external periphery of the cover body 1202.

As shown in FIG. 1B, when the syringe 1100 is connected to the lure lock coinfusion port 1200, the person implementing the connection is able to simply insert the second lure part 1141 into the slit with the slit of the valve 1204 into which the second lure part 1141 is to be inserted visible, by moving the locknut 1130 up to the first lure part 1144 to expose the second lure part 1141.

This can also be used as a lure slip syringe and lure slip coinfusion port with the second lure part 1141 inserted in the slit. However, to enhance the holding force of the connection, the person implementing the connection can further securely fix the syringe 1100 in the lure lock coinfusion port 1200 by lowering the locknut 1130 down to the second lure part 1141, and rotating the locknut 1130 to screw the locknut 1130 onto the thread groove 1203, as shown in FIG. 1C.

Since the locknut 1130 is moved to the second lure part 1141 where the fit is looser, and is freely rotatable, the operator can easily rotate the locknut 1130, even using one hand, so as to screw the locknut 1130 onto the thread groove 1203.
Moreover, even when the locknut 1130 is positioned on the first lure part 1144 where the fit is tighter, the fit is maintained at a level that enables the operator to rotate and move the locknut 1130 using only the fingers of one hand. This enhances the operability of the syringe in the medical field.

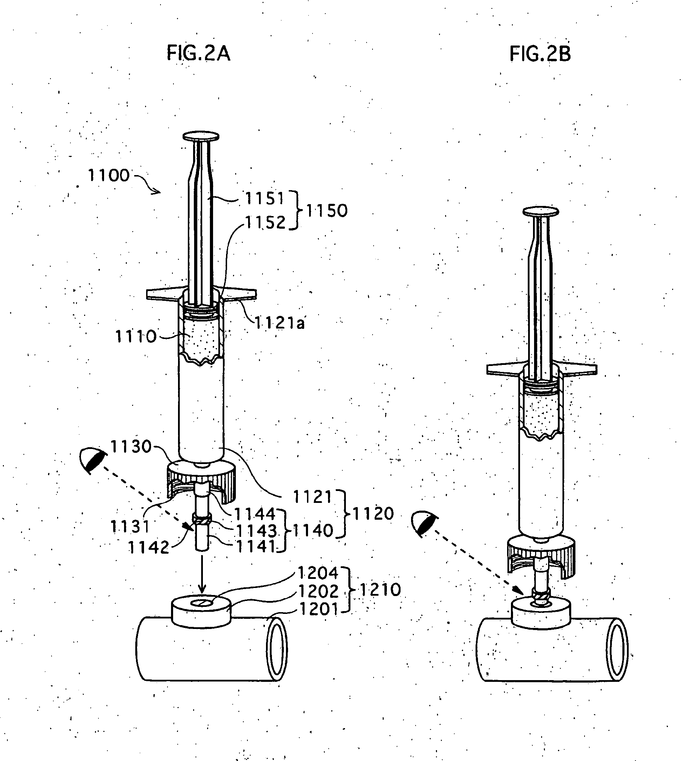

When the syringe 1100 is to be connected to the lure slip coinfusion port 1210 without the thread groove 1203 as shown in FIGs. 2A and 2B, the person implementing the connection can check the connection visually by inserting the second lure part 1141 into the slit of the valve 1204 after having moved the locknut 1130 to the first lure part 1144.

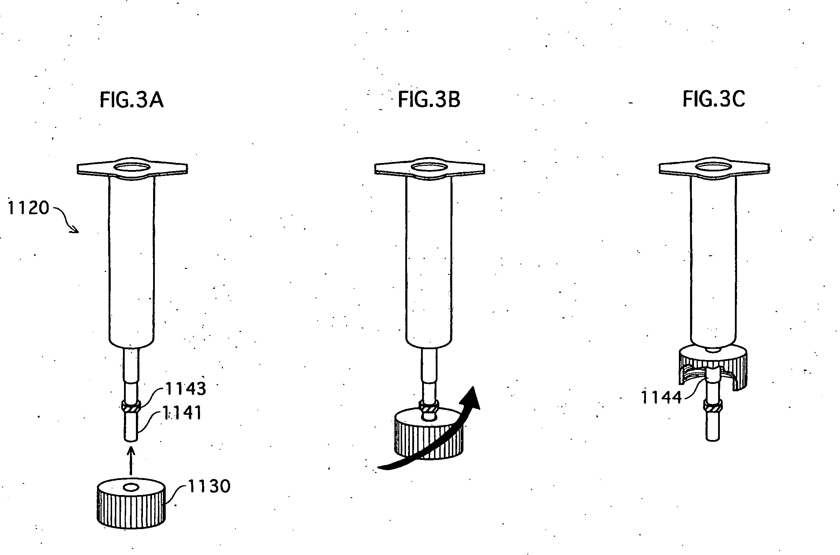

Since the fit of first lure part 1144 in the through hole is set to be moderately tight, the locknut 1130 is fixed in position, as shown in FIG. 3C, by moving the locknut 1130 to the upper end of the first lure part 1144.
Moreover, since the cover body 1202 and the locknut 1130 are distanced from one another when the stopper part is exposed, the two do not interfere with each other so as to prevent insertion of the syringe 1100, even if the external diameter of the cover body is larger than the internal diameter of the locknut 1130.

### Insertion of locknut over the protruding part

When attaching the locknut 1130 to the syringe part 1120, the extending part 1140 of the syringe part 1120 is inserted into the through hole of the locknut 1130.

In this case, the person doing the inserting can, as shown in FIGs. 3A and 3B, insert the locknut 1130 over the stopper 1143 without needing to apply a large force, by rotating the locknut 1130 in the direction of the spiral grooves provided on the periphery of the stopper 1143 while pushing on the locknut 1130.
Thus, with the syringe 1100 of the first embodiment, the locknut 1130 is moved to a position at which the second lure part 1141 is exposed and held so as not to drop under own weight, thereby enabling the person connecting the syringe to the lure slip coinfusion port 1210 to quickly insert the tip end of the second lure part 1141 into the slit of the valve 1204, similarly to the conventional lure slip syringe.

Even when connecting to the lure lock coinfusion port 1200, the operator quickly inserts the tip of the second lure part 1141 into the slit of the valve 1204, similarly to when connecting to the lure slip coinfusion port 1210, and subsequently securely fastens the syringe 1100 to the lure lock coinfusion port 1200 by moving the locknut 1130 down and tightening it on the thread groove 1203 of the lure lock coinfusion port 1200.

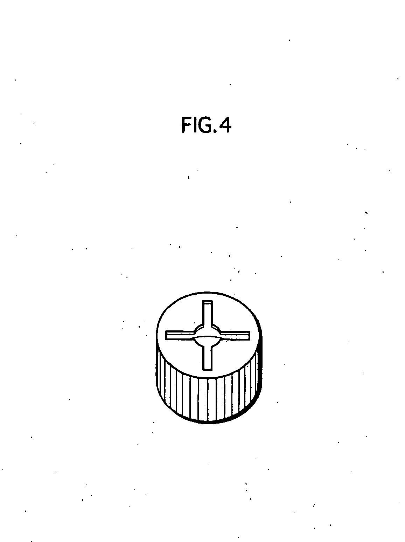

Note that in order to facilitate the insertion of the locknut 1130 over the extending part 1140, slits may be provided around the through hole of the locknut 1130 as shown in FIG. 4, making it easier to pass the locknut 1130 over the stopper 1143.
Although in the first embodiment spiral grooves are provided in the outer periphery of the stopper 1143, this is merely by way of example, and spiral grooves need not be provided on the stopper 1143.

### Second Embodiment

A syringe 1300 of the second embodiment is a pre-filled syringe which has been filled with a liquid medication 1110 and which can be quickly inserted and removed in relation to both lure lock coinfusion ports and lure slip coinfusion ports, similarly to the syringe 1100 of the first embodiment.

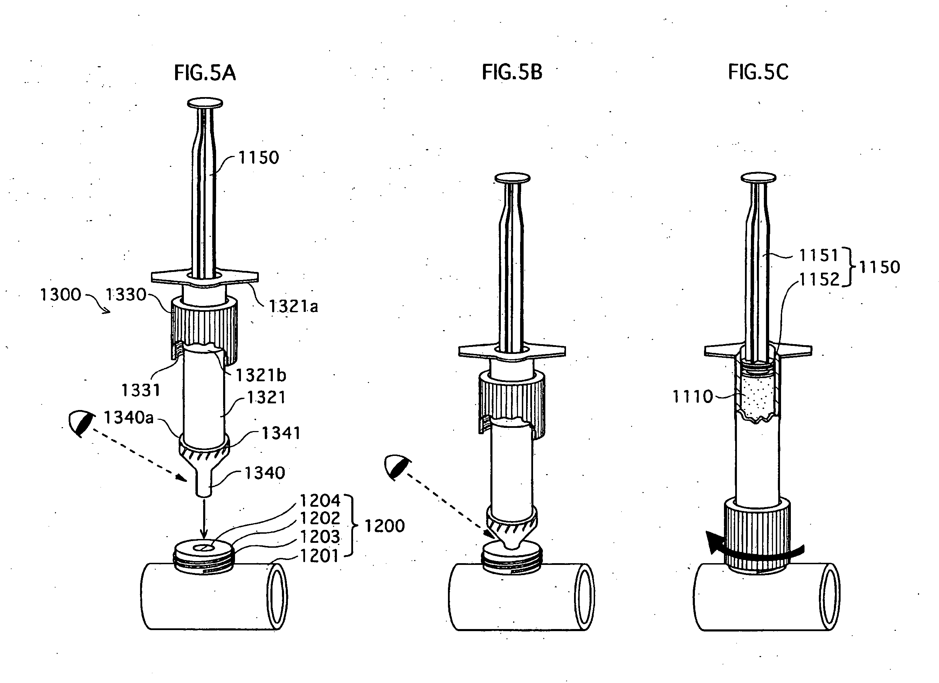

As shown in FIG. 5A, the syringe 1300 has a liquid medication 1110 filled in a tubular syringe body 1321 having a funnel-shaped lure 1340 disposed at a tip end thereof, is sealed by the plunger 1150, and has a cylindrical locknut 1330 installed on the syringe body 1321.
There is a stepped part 1340a at the boundary between the lure 1340 and the syringe body 1321, and spiral grooves 1341 are formed on the lure 1340 in proximity to the boundary.

The spiral grooves 1341 facilitate the insertion of the locknut 1330 over the syringe body 1321, similarly to the spiral grooves provided on the stopper 1143 in the first embodiment.
The locknut 1330 has a through hole provided therein, the diameter of which is d5.
The periphery of the syringe body 1321 has a large diameter part 1321b whose external diameter is larger than the external diameter of other parts. The external diameter of the large diameter part is d6, and the diameter of the other parts is d7.

The value of d6 is set such that the locknut 1330 can be moved easily by hand without falling under its own weight when the locknut 1330 is inserted onto the large diameter part 1321b
In short, the value of d6, is very close to, but a little larger than the value of d5.

The locknut 1330 is movable over the entire length of the syringe body 1321, and is stopped when it contacts a flange part 1321a and the stepped part 1340a, which exist at respective ends of the syringe body 1321.
As shown in FIG 5B, when the syringe 1300 is connected to the lure lock coinfusion port 1200, the person implementing the connection is able insert the lure 1340 into the slit easily, with the slit of the valve 1204 into which the lure 1340 is to be inserted visible, by moving the locknut 1330 up to the large diameter part 1321b.

With the lure 1340 inserted in the slit, use as a lure slip syringe and lure slip coinfusion port is possible. However, to enhance the holding force of the connection, the user implementing the connection can further securely fix the syringe 1300 in the lure lock coinfusion port 1200 by lowering the locknut 1330 down to the stepped part 1340a, and rotating the locknut 1330 to screw it onto the thread groove 1203, as shown in FIG. 5C.

Since the locknut 1330 is moved to a position on the syringe body 1321 where the fit is looser, and is freely rotatable, the operator can easily rotate the locknut 1330 even using one hand, so as to screw the locknut 1330 onto the thread groove 1203, as in the first embodiment.
Moreover, even when the locknut 1330 is positioned at a part of the syringe body 1321 where the fit is tighter, the fit is maintained at a level at which the user can rotate and move the locknut 1130 using only the fingers of one hand. This enhances the operability of the syringe in the medical field.

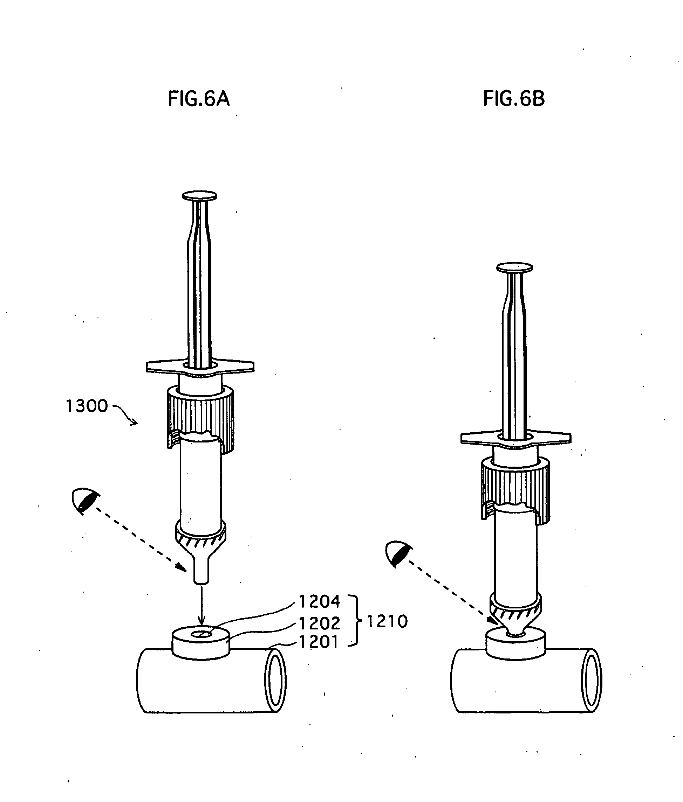

When the syringe 1300 is to be connected to the lure slip coinfusion port 1210 without a thread groove 1203 as shown in FIGs. 6A and 6B, the person implementing the connection can check the connection visually by inserting the lure 1340 into the slit of the valve 1204 after having move the locknut 1330 to the large diameter part 1321b.
Moreover, since the cover body' 1202 and the locknut 1330 are distanced from one another, they do not interfere with one another to prevent insertion of the syringe 1300, even if the external diameter of the cover body 1202 is larger than the internal diameter of the locknut 1330.

As described above, with the syringe 1300 of the second embodiment, the locknut 1330 is moved to a position at which the tip end of the lure 1340 is exposed and held so as not to drop under its own weight, thereby enabling the person connecting the syringe to the lure slip coinfusion port 1210, to quickly insert the tip of the lure 1340 into the slit of the valve 1204, similarly to the conventional lure slip syringe.

Here, the position at which the tip of the lure is exposed refers to a position for the locknut 1330 at which a person connecting the syringe 1100 and the lure lock coinfusion port 1200 or lure slip coinfusion port 1210 in a normal working position is able to see the tip end of the lure 1340.
Even when connecting to the lure lock coinfusion port 1200, the operator quickly inserts the tip of the lure 1340 into the slit of the valve 1204, similarly to when connecting to the lure slip coinfusion port 1210, and subsequently securely fastens the syringe 1300 to the lure lock coinfusion port 1200 by moving the locknut 1330 towards the stepped part 1340a, and tightening it on the thread groove 1203 of the lure lock coinfusion port 1200.

Although in the second embodiment, spiral grooves 1341 are provided on the lure 1340, this is merely by way of example and spiral grooves need not be provided in the lure 1340.
Note also that although in the first and second embodiments the locknut 1130 of the syringe 1100 and the locknut 1330 of the syringe 1300 are nuts that engage with implements such as the coinfusion port, this is merely by way of example, and this part need not be a nut. For instance, a lock part 1132 having a claw part 1133a may be used instead of a nut.

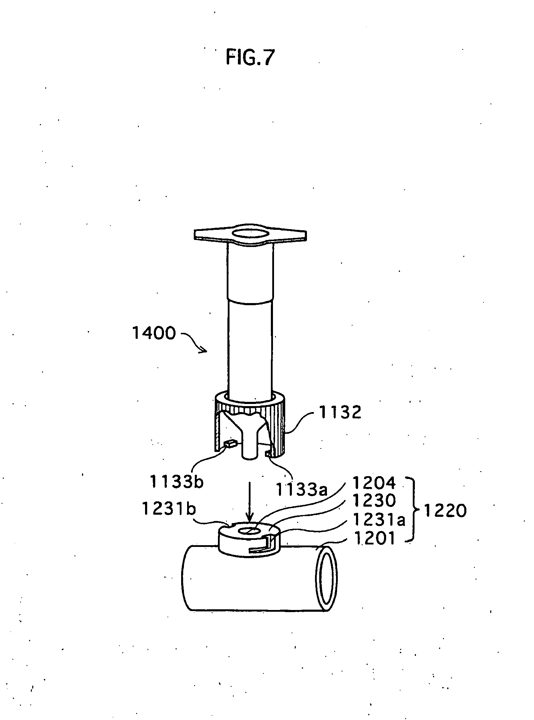

In this case, the change in the locking mechanism of the syringe necessitates a change in the structure of the engaging part of the lure lock coinfusion port, an example of which is a coinfusion port 1220, which has L-shaped grooves 1231a, as shown in FIG. 7.

### Third Embodiment

### Structure

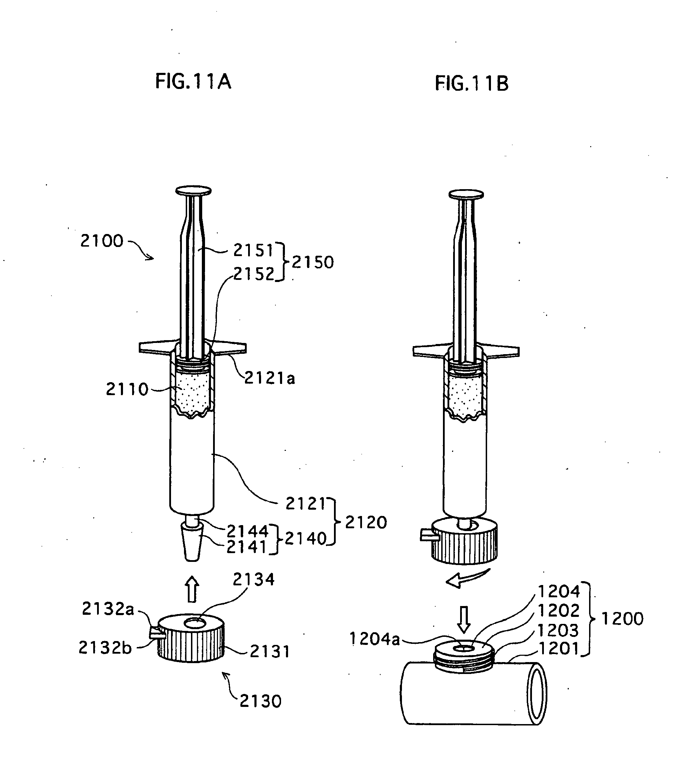

FIGs. 11A and 11B illustrate the syringe of the third embodiment.

A syringe 2100 is a pre-filled syringe that has been filled with a liquid medication 2110 and can be quickly inserted and removed. from in relation to both lure lock coinfusion ports and lure slip coinfusion ports.
The lure lock coinfusion port is, for instance, the lure lock coinfusion port 1200 shown in FIG. 11B, and consists of the rubber valve 1204 having a slit 1204a and held in place by the cylindrical cover body 1202 on the side of the tube body 1201 that forms the transfusion line or similar flow pass. The thread groove 1203 is provided on the external periphery of the cover body 1202.

The lure slip coinfusion port refers to a coinfusion port such as the coinfusion port 1200 from which the thread groove 1203 has been omitted.
The syringe 2100 is formed, as shown in FIG. 11A, by sealing a tubular syringe part 2120 with a plunger 2150 after filling the syringe part 2120 with the liquid medication 2110, and rotatably attaching a lock part 2130 (as an exemplary connection supporting member) to one end of the syringe part 2120.

Consequently, an operator is able to rotate the lock part 2130 easily even with one hand, and screw the lock part 2130 onto the thread groove 1203.
The syringe part 2120 is formed from a tubular lure 2140 extending from one end (hereinafter referred to as "the first end") of a tubular syringe body 2121, and a flange 2121a provided at the opposite end to the first end (hereinafter referred to as "the second end").

The lure 2140 is formed from a tubular first lure part 2144, which has a diameter d1 and is located towards a base of the lure 2140, and a second lure part 2141, which is a tapered members located towards the tip of the lure. The diameter d2 of the tip of the second lure part 2141 is small, and the diameter d3 of the other end is the largest diameter anywhere on the lure 2140. With this arrangement, d3 > d2 and d3 > d1.
The lock part 2130 is a detachable locking mechanism for strengthening the connection between the syringe 2100 and a connecting implement such as a port, and consists of a slider plate 2132 inserted into a cylindrical lock body 2131 with a base.

The lock body 2131 has a thread provided in the cylindrical internal surface thereof, a through hole 2134 of diameter d4 provided in the base, and a rectangular opening 2131a provided in the cylindrical side. Further, the slider plate 2132 is movably inserted into the opening 2131a.
Note that d4 is larger than d3 to enable the second lure part 2141 to pass through the through hole 2134.

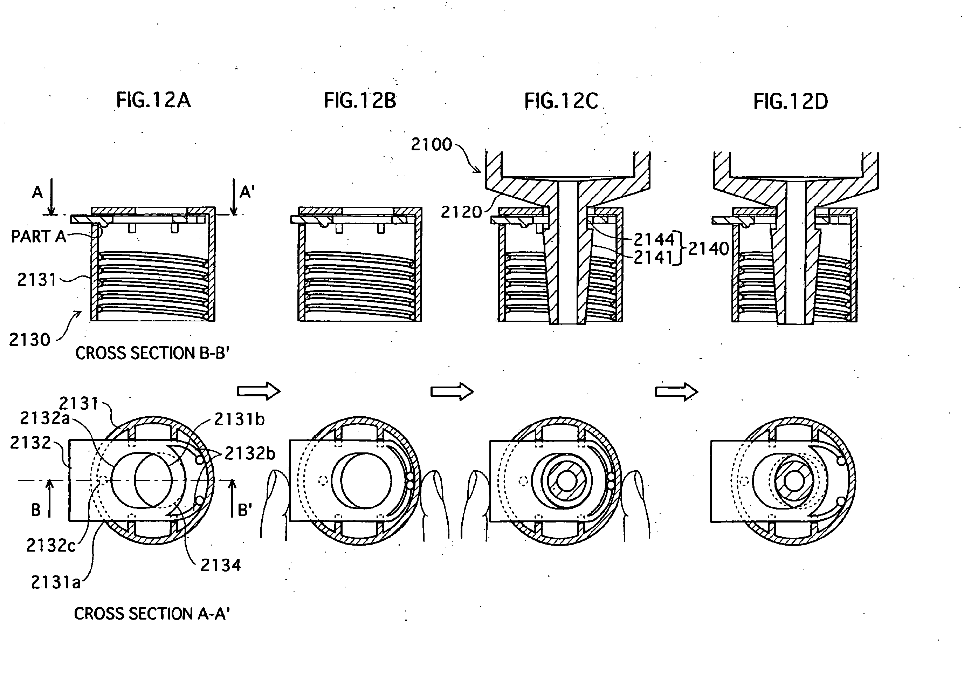

The slider plate 2132 engages the lock part 2130 and the lure 2140, and as shown in FIG. 12A, is provided with a substantially central through hole 2132a, and two elastically-deforming rod-shaped elastic parts 2132b at a front end, the tips of which bend inwardly. A protrusion 2132c is provided whereby part of the surface is raised near the posterior end of the slider plate 2132.

### Method for attaching the lock part

As shown in FIG 12A, the slider plate 2132 experiences a force that attempts to push it out through the opening 2131a (leftwards in the drawing) due to the elastic forces of the elastic parts 2132b, and is held stationary by the protrusion 2132c contacting an internal surface portion A of the lock body 2131.

Inthecross-sectionA-A', aplan-viewholeformedbythethrough hole 2134 of the lock body 2131 overlapping the through hole 2132a of the slider plate 2132 is small enough to prevent the second lure part 2141 from passing.
As shown in FIG. 12B, when the slider plate 2132 is squeezed with the fingers, the elastic parts 2132b elastically deform and the slider plate 2132 enters into the lock body.

0080 The plan-view hole 2131b formed by the through hole 2132a of the slider plate 2132 overlapping the through hole 2134 of the lock body 2131 becomes large enough to pass the second lure part 2141, as shown in FIG. 12C.
When the fingers removed after the second lure part 2141 has been inserted through the plan-view hole 2131b, the slider plate 2132 is returned to its original position by the restoring force of the elastic parts 2132b, as shown in FIG. 12D.

The tubular first lure part 2144 whose diameter is small fits into the plan-view hole 2131b. Thus, as a consequence of the first lure part 2144 being held between the slider plate 2132 and the lock body 2131, the lock part 2130 is rotatably attached to the syringe in the cylindrical direction.
Further, the lock part 2130 can be detached from the syringe part 2120 by executing the above operations in reverse order.

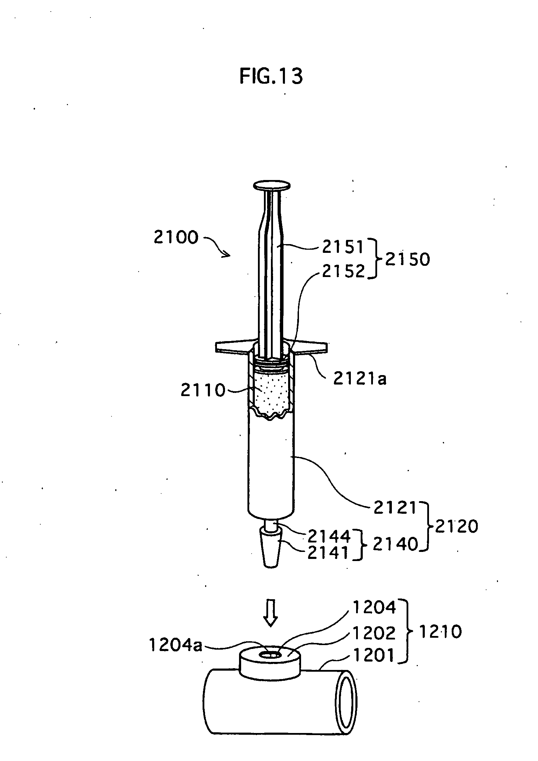

When connecting the syringe 2100 to the lure lock coinfusion port 1200, attaching the lock part 2130 to the syringe part 2120 enables the syringe 2100 to be connected without difficulty, similarly to conventional lure lock syringe, as shown in FIG. 11B.
Further, when connecting the syringe 2100 to the lure slip coinfusion port 1210 as shown in FIG. 13, removing the lock part 2130 from the syringe part 2120 enables the syringe 2100 to be connected without the lock part 2130 interfering with the lure slip coinfusion port 1210, in the same problem-free way as a conventional lure slip syringe.

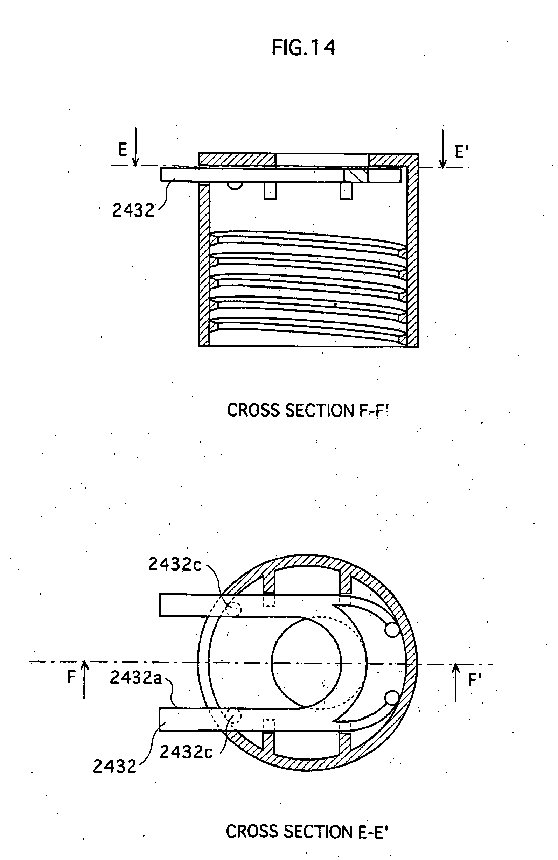

As described above, with the syringe 2100 of the third embodiment, the lock part 2130 can be easily detached by squeezing and moving the slider plate 2132 using the fingers, enabling the person performing the connection to switch between lock-attached and lock-removed configurations depending on the type implement to which the syringe 2100 is to be connected.
In the third embodiment, the through hole 2132a is provided in the slider plate 2132, although another shape may be used rather than the through hole. For instance, a U-shaped cut-out 2432a may be formed in place of the through hole 2132a, as in a slider plate 2432 of FIG. 14. In this case, a member is not present at the position of the protrusion 2132c so it is necessary to provide protrusions 2432c.at positions where members are present. In FIG. 14, protrusions 2432c are provided at two positions located symmetrically about a central axis, so as to share the force exerted on the slider plate.

In the third embodiment, the locking mechanism of the lock part 2130 is realized by a thread provided therein. However the lock part 2130 need not include a thread, and may instead include a structure such as a claw or a protrusion as an engagement means.
Moreover, in the third embodiment the through hole 2134 and the slider plate 2132 are provided in the lock part 2130, and the second lure part 2141 whose diameter is locally reduced, on the lure 2140. However, a similar effect is achieved to the syringe 2100 of the third embodiment even when members for engaging the lock part 2130 and the syringe part 2120 are interchanged between the lock part 2130 and the lure 2140.

### Fourth Embodiment

### Structure

A syringe 2500 of the fourth embodiment can be quickly inserted and removed in relation to both lure lock coinfusion ports and lure slip coinfusion ports similarly to the syringe 2100 of the third embodiment, but differs in the structure of the lock part.

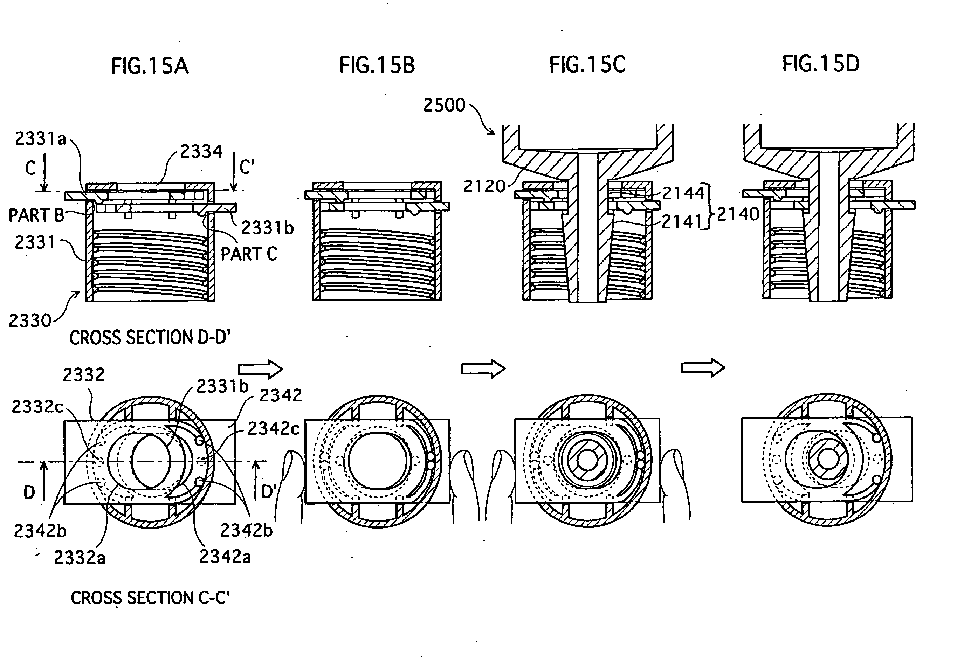

As shown in FIG. 15A, a lock part 2330 is a detachable locking mechanism for strengthening the connection between the syringe 2500 and a connecting implement, and includes a slider plate 2332 and a slider plate 2342 inserted into a cylindrical lock body 2331 with a base.
The lock body 2331 is provided with a thread groove in the internal surface thereof, a through hole 2334 of diameter d5 in a central region of the base, and a rectangular opening 2331a and a rectangular opening 2331b in respective sides.

The slider plate 2332 and the slider plate 2342 are movably inserted into the opening 2331a and the opening 2331b, respectively.
Note that the value of d5 of the through hole 2334 is larger than the posterior end diameter d3 of the second lure part 2141 to enable the second lure part 2141 to pass through the through hole 2334.
The slider plate 2332 and the slider plate 2342 act to engage the lock part 2330 and the lure 2140, and as shown in FIG. 15A, respectively include a substantially central through hole 2332a and a substantially central through hole 2342a. Each of the slider plate 2332 and the slider plate 2342 includes two elastically-deforming rod-shaped elastic parts at one end, the tips of which bend inwardly. The slider plate 2332 is provided with two elastic parts 2332b, and the slider plate 2342 is provided with two elastic parts 2342b. A protrusion 2332c is provided on the slider plate 2332 and a protrusion 2342c is provided on the slider plate 2342, whereby part of the surface of each slider plate is raised near the opposite end to the elastic parts.

### Method for attaching the lock part

As shown in FIG 15A, the slider plate 2332 experiences a force that attempts to push it out through the opening 2331a due to the restoring forces of the elastic parts 2332b. Likewise, the slider plate 2342 experiences a force that attempts to push it out through the opening 2331b due to the restoring forces of the elastic parts 2342b. The slider plate 2332 and the slider plate 2342 are held stationary in the lock body 2331 by the protrusion 2332 and the protrusion 2342 respectively contacting internal surface portions B and C of the lock body 2331.

In the cross-section C-C' a plan-view hole formed by the through hole 2332a of the slider plate 2332 overlapping the through hole of the 2342a of the slider plate 2342 is small enough to prevent the second lure part 2141 passing.
As shown in FIG. 15B, when the slider plate 2332 and the slider plate 2342 are simultaneously squeezed with the fingers, the elastic parts 2332b and the elastic parts 2342b elastically deform, and the slider plate 2332 and the slider plate 2342 move into the lock body 2331.

The plan-view hole formed by the through hole 2332a of the slider plate 2332 overlapping the through hole 2342a of the slider plate 2342 becomes large enough to pass the second lure part 2141, as shown in FIG. 15C.
When the fingers are removed after the second lure part 2141 has been inserted through the plan-view hole, the slider plate 2332 and the slider plate 2342 are returned to their original positions by the restoring forces of the elastic parts 2332b and the elastic parts 2342b, as shown in FIG. 15D.

A tubular first lure part 2344 whose diameter is small fits the plan-view hole. Thus, as a consequence of the first lure part 2144 being held between the slider plate 2332 and the slider plate 2342, the lock part 2330 is rotatably attached to the syringe part 2120 in the cylindrical direction.
An effect of the present invention that an operator can rotate the lock part 2330 easily, even using only one hand, and screw the lock part 2330 onto the thread groove.

Further, the lock part 2330 can be detached from the syringe part 2120 by executing the above operations in reverse order.
As described above, when connecting the syringe 2500 to the lure lock coinfusion port 1200, attaching the lock part 2330 to the syringe part 2120 enables the syringe 2500 to be securely connected without difficulty, similarly to a conventional lure lock syringe.

Further, when connecting the syringe 2500 to the lure slip coinfusion port 1210, removing the lock part 2330 from the syringe part 2120 enables the syringe the syringe 2500 to be connected, similarly to a conventional lure slip syringe, without the difficulty of the lock part 2330 interfering with the lure slip coinfusion port 1210.
In the fourth embodiment, the through hole 2332a and the through hole 2342a are respectively provided in the slider plate 2332 and the slider plate 2342, although through holes need not be used. For instance, U-shaped cut-outs of the type described in the third embodiment may be formed in place of the through holes.

In the fourth embodiment, the locking mechanism of the lock part 2330 is realized by a thread provided therein. However the lock part 2330 need not use a thread, and may instead include a structure such as a claw or a protrusion as engagement means, as described in the third embodiment.
Moreover, in the fourth embodiment, the slider plate 2332 and the slider plate 2342 are provided in the lock part 2330, and the second lure part 2141 whose diameter is locally reduced is provided on the lure 2140. However, a similar effect is achieved even when the members for engaging the lock part 2330 and the syringe part 2120 are interchanged between the lock part 2130 and the lure 2140.

### Fifth Embodiment

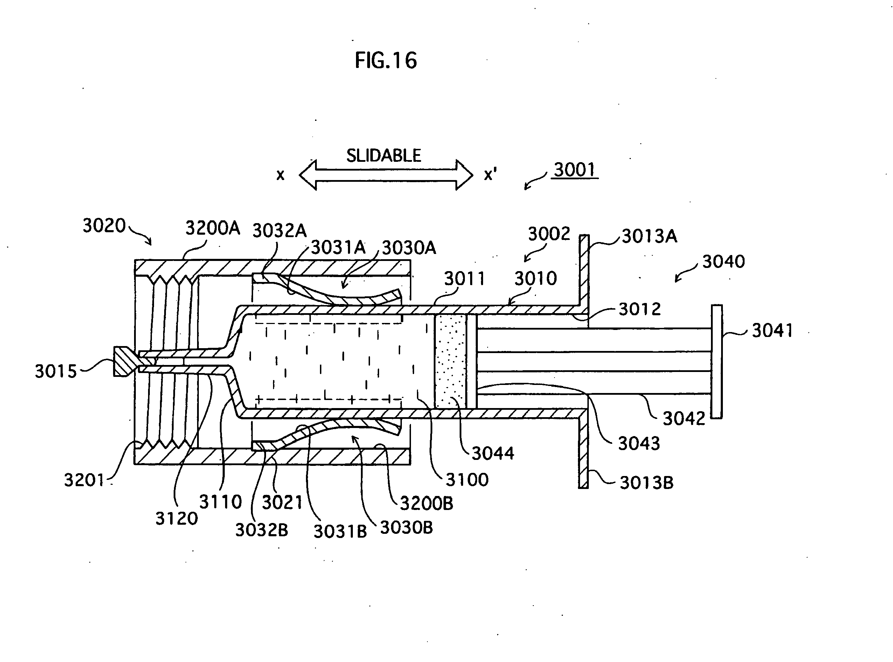

FIG. 16 is a cross-sectional drawing showing the construction of a lock connector-attached syringe of the fifth embodiment of the present invention. For ease of description, a plunger 3040 is shown here in normal side elevation rather than in cross-section.
The construction of the lock connector-attached syringe shown in FIG. 16 is broadly divided into a pre-filled syringe 3002 and a lock connector 3020 as a connection supporting member.

The pre-filled syringe 3002 is constituted from from a syringe body 3010, a plunger (also known as a piston) 3040, and the like.
The syringe body 3010 is a tubular body formed by injection molding a material with excellent chemical resistance, such as polyethylene, poly-propylene, or poly-carbonate. The tip end of the syringe body 3010 is sealed by a top face part 3110, and a hollow lure 3120 juts out from the center of the top face part 3110. The lure 3120 is processed by drawing to give a taper shape in compliance with ISO6/100 so that a regular needle hub 3060 can be attached easily. In FIG. 16, a cap 3015 is attached to the tip of the lure 3120.

On the other hand, an opening 3012 is formed at the posterior end of the syringe body 3010 and a pair of flanges 3013A and 3013B are formed so as to surround the opening.
In the following, a direction parallel to a longitudinal direction of the syringe body 3010 is referred to as the "axial direction" and a direction orthogonal to the axial direction as the "radial direction". Moreover, a direction parallel with the tip of the syringe is referred to as the x direction and a direction parallel with the posterior end of the syringe is referred to as the X' direction.

The plunger 3040 is composed of a resin material with excellent chemical resistance, similarly to the syringe body 3010, and includes a plunger body 3042 having a cruciform profile for purposes of reinforcement, and at each end of the body, disk-shaped end pieces having main surfaces in the radial direction. One of these end pieces is a pressing end part 3041 for pressing by an operator, and the other end piece is a head part 3043 that is inserted into the syringe body 3010 in the axial direction.

Packing 3044 composed of an elastomer material is disposed at the tip of the head part 3043 so as to make a tight contact with the internal walls of the syringe body 3010. In the drawing, the tip of the packing 3044 is flat. However, the packing 3044 may be slightly conical to enable a sealed contact with the inside of the top face part 3110 of the syringe body 3010. Here, medication 3100 is held in the syringe body 3010, which is internally sealed by the packing 3044 and the cap 3015.

When using the pre-filled syringe 3002 of this type of construction, the operator removes the cap 3015 to enable discharge of the medication 3100. Then, hooking fingers around the pair of flanges 3013A and 3013B, the operator pushes the pressing end part 3041 of the plunger 3040 into the syringe body 3010 with a thumb or the like, causing the medication 3100 to be discharged from the tip of the lure according to the depressed amount of the plunger.

The lock connector-attached syringe of the fifth embodiment is characterized by the insertion of the lock connector 3020 as a cloaking body that is slidable in the axial direction on the external surface 3011 of the syringe body 3010. The lock connector 3020 is used in the medical field as a connecting implement for connecting a syringe to a fixed connection-type port in a transfusion line system or blood collection line system.

Specifically, the lock connector 3020 includes a cylindrical connector body 3021, as shown in the FIG. 16 cross-sectional drawing.
The connector body 3021 is formed by injection molding a resin material substantially similar to that used in the syringe body 3010 in a cylindrical shape. A thread part 3201 screwable to an external port is formed on an internal surface 3200B at the tip end of the connector body 3021. Further, a saddle spring body 3030A and a saddle spring body 3030B, which are not part of the connector body 3021, are joined to the connector body 3021 at a central region of the internal surface 3200B.

The saddle spring body 3030A and saddle spring body 3030B are made by forming saddle-shapes from a material of a moderate strength and elastic restoring, strength, such as poly-propylene, or poly-ethylene, and are securely connected to the connector internal surface 3200B using an adhesive or the like to form a connecting part 3032A and a connecting part 3032B at ends thereof. The other ends of the saddle spring body 3030A and saddle spring body 3030B curve away from a central axis direction of the connector body 3021. This enables portions other than the connecting part 3032A and connecting part 3032B to form a spring 3031A and a spring 3031B, with the connecting part 3032A and connecting part 3032B as fulcrums. The saddle spring body 3030A and saddle spring body 3030B are symmetrically disposed in the axial direction, on the internal surface 3200B, and the shortest distance between opposing surfaces of the spring 3031A and spring 3031B is set to be smaller than the external diameter of the syringe body 3010.

Note that although a construction using the saddle spring body 3030A and saddle spring body 3030B has been described here, the present invention is not limited to this construction. Two or more saddle spring bodies may be provided, or a cylindrical spring body matching the internal diameter of the connector body 3021 may be fitted to the internal surface 3200B and the syringe body 3010 held in place as a result of the cylindrical spring body circumferentially contacting external surface 3011 of the syringe body 3010. Alternatively, the saddle spring body 3030A and saddle spring body 3030B may be integrated with the connector body 3021. Further, band-shaped board springs may be used instead of the saddle spring body 3030A and saddle spring body 3030B. However, in terms of securing a contact surface with the syringe body 3010, it is preferable to use curved spring bodies, such as the saddle shaped spring body 3030A and saddle spring body 3030B, which have a large area in contact with external surface 3011 of the syringe body 3010.

In the fifth embodiment, by appropriately positioning the saddle spring body 3030A and saddle spring body 3030B within the connector body 3021, the syringe body 3010 is gripped between the spring 3031A and spring 3031B in the saddle spring body 3030A and saddle spring body 3030B when the syringe body 3010 is inserted into the connector body 3021. Moreover, the connector body 3021 can be slid back and forth in the axial direction (X-X' direction), while being held by the elastic restoring force of the saddle spring body 3030A and saddle spring body 3030B so as to be not easily separated from the syringe body 3010. The elastic restoring force of the spring 3031A and spring 3031B on the syringe body 3010 can be adjusted by various methods. These include using a hard material for the saddle spring body 3030A and saddle spring body 3030B, using a soft material but increasing the degree of adhesion on the syringe body 3010, and setting the curvature of the spring 3031A and spring 3031B such that the minimum distance between the spring 3031A and spring 3031B is reduced.

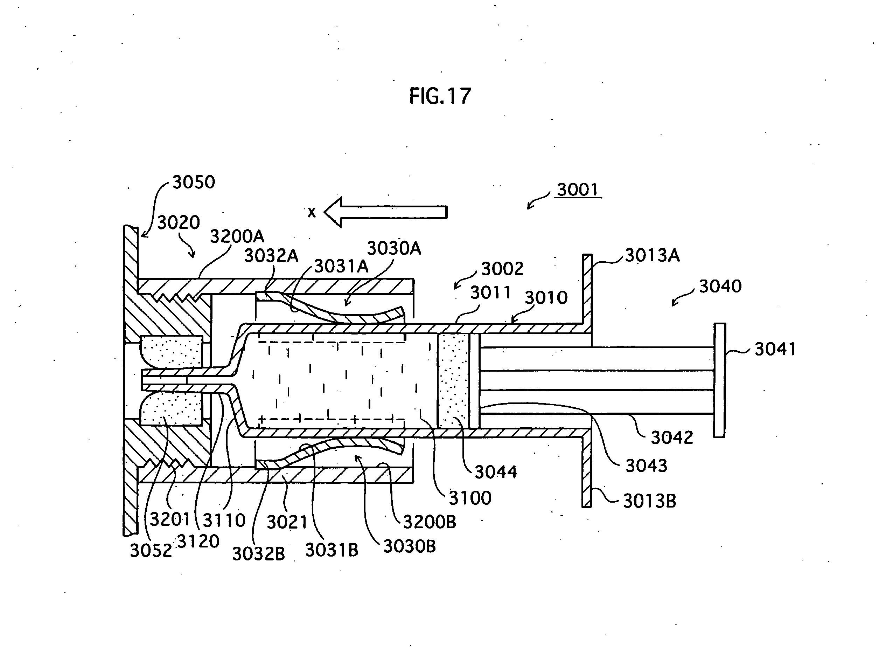

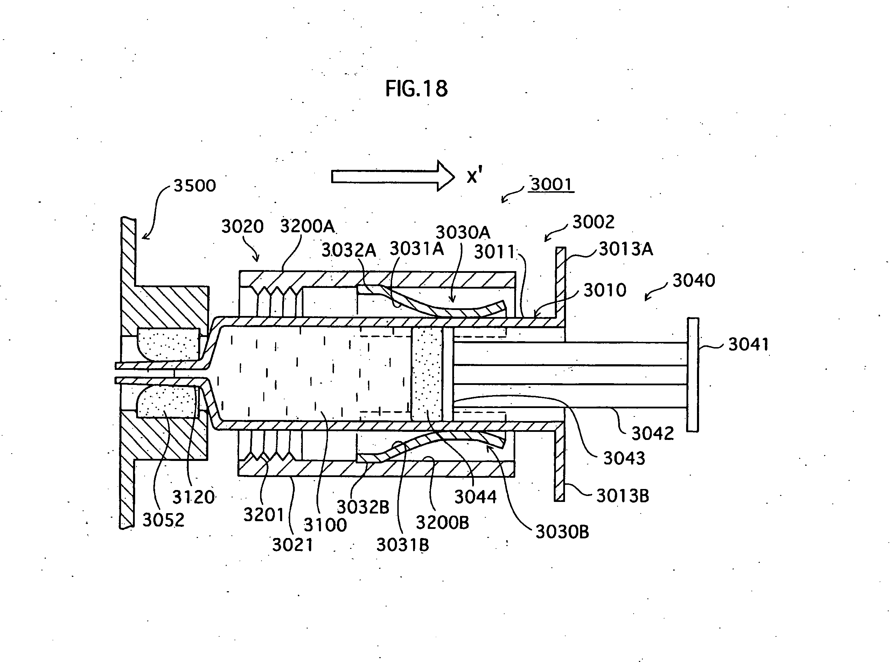

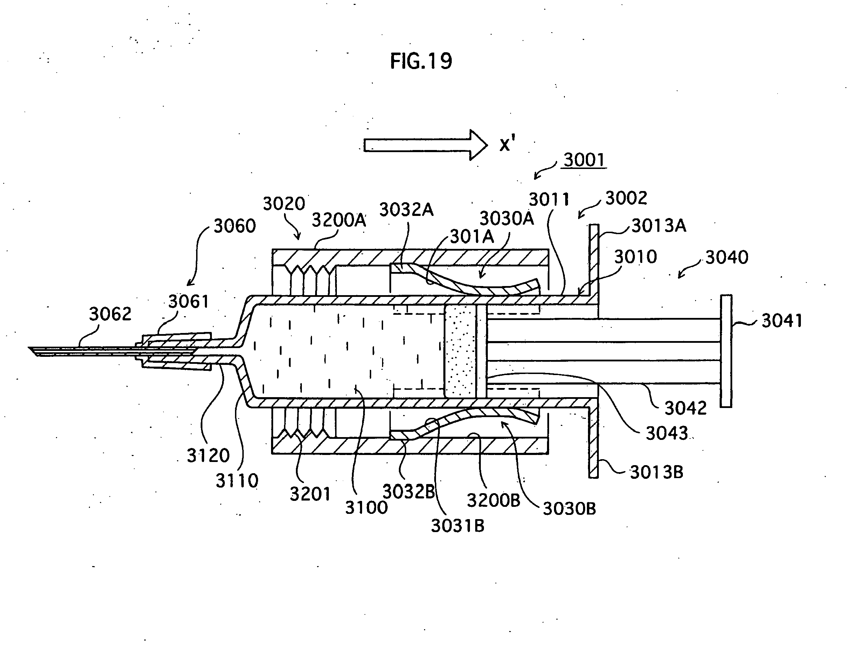

Note that once the connector body 3021 has been fitted onto the syringe body 3010,subsequent separation is unnecessary.
The following describes the effects of the syringe body 3010 and the lock connector 3020 as features of the fifth embodiment. FIG. 17 shows a construction of the lock connector attached syringe connected to a fixed connection-type port 3050 of a transfusion line system. FIG. 18 shows a construction of the lock connector attached syringe connected to a direct connection-type port 3500 of a transfusion line system. Further, FIG. 19 shows the construction of the lock connector attached syringe connected to the needle hub 3060.

With a lock connector-attached syringe 3001 having the construction shown in FIG . 16, as a first step, the operator grasps an external surface 3200A and slides the connector body 3021 towards the tip of the syringe (X direction) while holding the syringe body 3010 with their fingers. By doing so, the operator can move the thread part 3201, which is formed in the internal surface 3200B of the connector body 3021, to a position corresponding to the lure 3120 of the syringe body 3010.

Further, utilizing this state, as a second step, if the operator screws the thread part 3201 of the connector body 3021 onto the fixed connection-type port 3050, the lure 3120 of the syringe body 3010 can be tightly inserted into packing 3052 of the port while the syringe body 3010 is securely held by the elastic restoring force in the saddle spring body 3030A and saddle spring body 3030B within the lock connector 3020. Here, the operator is able to speedily perform the screwing operation while holding the syringe body 3010 in one hand. Hence, the operator is able, for instance, to leave the pre-filled syringe 3002 connected to a port 3050 for long periods, and administer the medication 3100 in required amounts into the port 3050 by appropriately pressing the plunger 3040 into the syringe body 3010 with a high degree of stability. Because the syringe body 3010 is held between the saddle spring body 3030A and saddle spring body 3030B, the syringe body 3010 will not be mistakenly removed from the port 3050, even if some force is applied to the syringe body 3010.

On the other hand, it is also possible to perform the following operations using the syringe body 3010 and the lock connector 3020. Specifically, if the operator grasps the external surface 3200A and slides the connector body 3021 toward the posterior end of the syringe (X' direction) in a direction opposite to that described above while holding the syringe body 3010 with their fingers, the connector body 3021 slides over external surface 3011 of the syringe body 3010, and the lure 3120 is exposed to the exterior, as shown in FIG. 18.

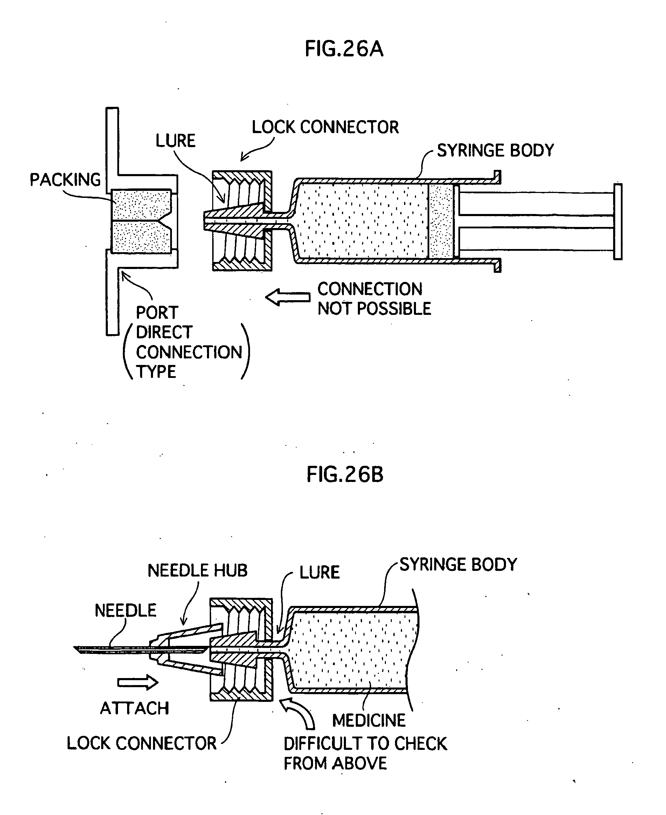

In the conventional example shown in FIG. 26A, the locknut interferes with the direct connection-type port 3500, preventing the connection of the lure 3120 to the port 3050. The lock connector 3020, however, can be kept on the syringe body 3010 so as not to interfere, and it is therefore possible to tightly insert the lure 3120 of the syringe body 3010 into the packing 3052 of the direct connection-type port 3500, while maintaining an excellent visual check on the lure 3120 of.the syringe body 3010.

Moreover, having the lure 3120 exposed to the exterior in this way also makes it possible to attach the needle hub 3060 as shown in FIG. 19. The needle hub 3060 has a construction in which a socket 3061, which is composed of a resin material and formed to match the shape of the lure 3120; holds a needle tube 3062 as a so-called injecting needle. With the conventional syringe in which the lock connector is fixed on the lure and cannot be detached as shown in FIG. 26B, the lock connector obstructs the view of the operator and makes it difficult to visually check on the positioning of the lure and the needle hub 3060, thereby creating the danger of the operator mistakenly pricking himself and coming into contact with infectious material. In the fifth embodiment, however, slidably positioning the lock connector 3020 enables easy visual checking of the lure 3120, and consequently, the needle hub 3060 can be attached simply.

Here, since' the described operations of the lock connector-attached syringe 3001 can easily (with just one hand if well practiced) be executed in reverse, the lock connector 3020 can be slidably positioned on the syringe body 3010 depending on whether or not it is required, to enable the medication 3100 to be administered after choosing the most operable posture.

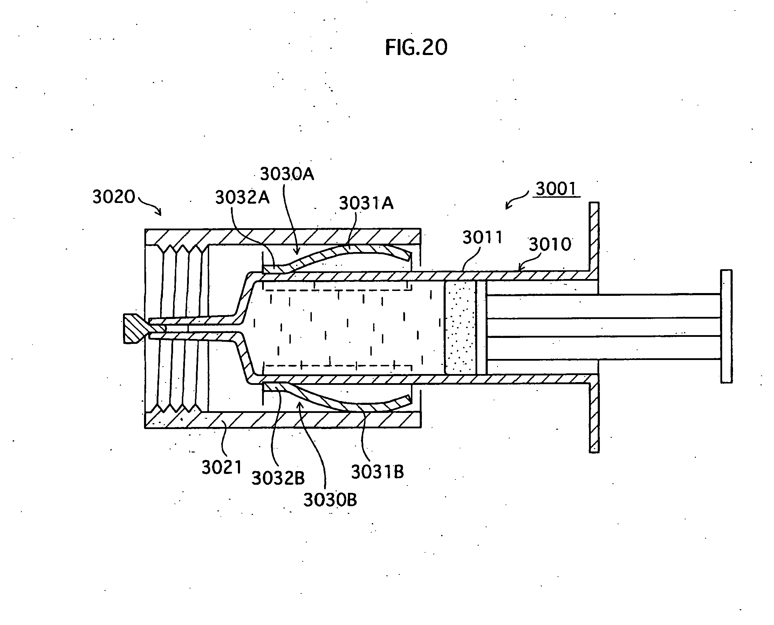

Note that although the fifth embodiment describes a construction in which the saddle spring body 3030A and saddle spring body 3030B are diposed on the internal surface 3200B of the lock connector, the present invention may have a construction in which the saddle spring body 3030A and saddle spring body 3030B are instead provided on the syringe body 3010 as shown in FIG. 20, and contact is maintained by the elastic restoring force of the spring 3031A. and spring 3031B pressing on the lock connector 3020.

### Sixth Embodiment

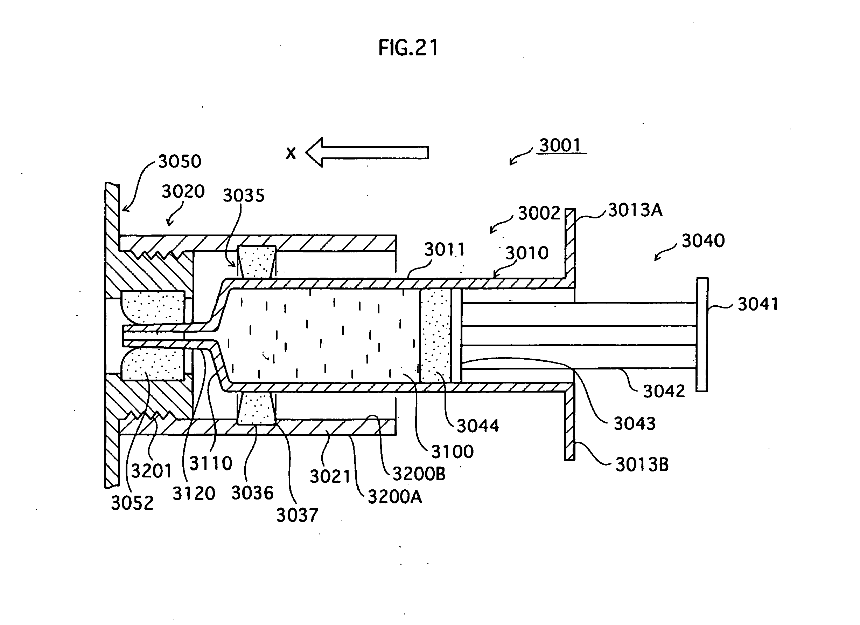

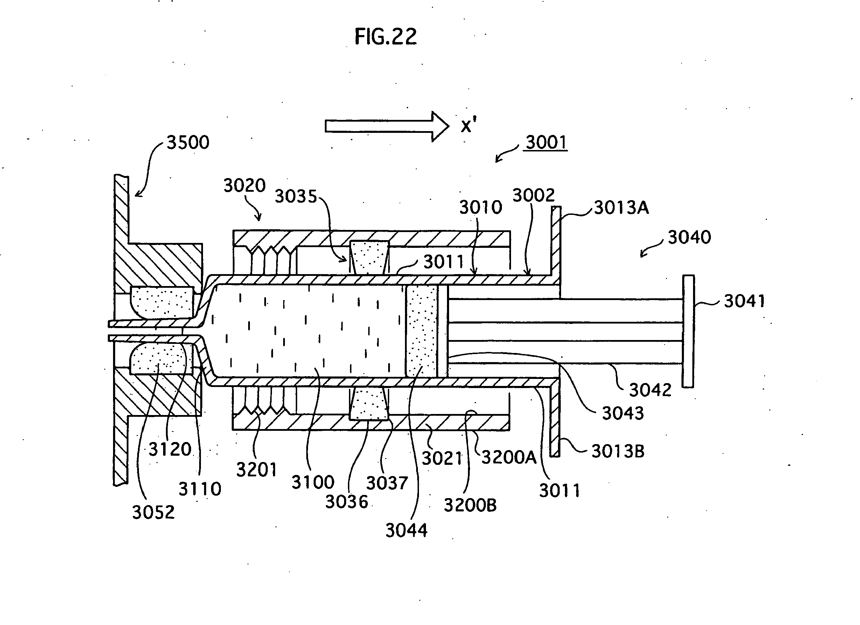

FIG. 21 shows a construction of a lock connector attached syringe connected to a fixed connection-type port 3050 of a transfusion line system. FIG. 22 shows a construction of a lock connector attached syringe connected to a direct connection-type port 3500 of a transfusion line system.
In the fifth embodiment, a construction was described in which the saddle spring body 3030A and saddle spring body 3030B were appropriately arranged within the connector body 3021, and the elastic restoring force of the saddle spring body 3030A and saddle spring body 3030B held the syringe body 3010. The sixth embodiment differs in that an elastomer ring 3035 is used instead of the saddle spring body 3030A and saddle spring body 3030B. Otherwise, the construction of the sixth embodiment is similar to that of the fifth embodiment.

Specifically as shown in FIG. 21, the connector body 3021 of the sixth embodiment has fitted therein the elastomer ring 3035 composed of a flexible rubber material that has excellent wear-resistance. The elastomer ring 3035 has a substantially rectangular profile (here it is trapezoidal), an external diameter set to be a little larger than the internal diameter of the connector body 3021, and an internal diameter set to be a little smaller than the external diameter of the syringe body 3010. An external periphery surface of the elastomer ring 3035 is fused or adhered as an engaging part 3037, to the internal surface 3200B of the connector body 3021 such that the elastomer ring 3035 does not easily separate from the connector body 3021.

The lock connector 3020 having this construction is slidably attached in the axial direction (X-X' direction) as a result of the elastomer ring 3035 gripping the syringe body 3010, and is thereby attached to the syringe body 3010 and slidable in the axial direction. Here, a constant frictional force is generated between the external surface 3011 of the syringe body 3010 and the internal surface 3036 of the elastomer ring 3035, and the syringe body 3010 is held in place both by this frictional force and by an elastic contraction of the elastomer ring 3035.

Thus, during use, the operator grasps the external surface 3200A while holding the syringe body 3010 with their fingers, and slides the connector body 3021 towards the tip of the syringe (X direction). The operator can then slide the thread part 3201, which is formed in the internal surface 3200B of the connector body 3021, to a position corresponding to the lure 3120 of the syringe body 3010, as shown in FIG. 21. Then, by using the structure in this state, and screwing the thread part 3201 of the connector body 3021 onto the fixed connection-type port 3050, the operator can tightly insert the lure 3120 of the syringe body 3010 into the packing 3052 of the port while having the syringe body 3010 held by the elastic restoring force and frictional force of the elastomer ring 3035 in the lock connector 3020. Here, similarly to the fifth embodiment, the operator doing the screwing is able to speedily perform the screwing operation while holding the syringe body 3010 in one hand. Hence, similarly to the fifth embodiment, the operator can leave the pre-filled syringe 3002 connected to the fixed connection-type port 3050, and press the plunger 3040 an appropriate amount into the syringe body 3010 with a high level of stability, to administer a required amount of the medication 3100 into the fixed connection-type port 3050.

On the other hand, in the sixth embodiment, if the operator grasps the external surface 3200A while holding the syringe body 3010 with their fingers, and slides the connector body 3021 towards the syringe posterior end (X' direction) in a direction opposite to that described above, the connector body 3021 slides over the external surface 3011 of the syringe body 3010 to expose the lure 3120, as shown in FIG 22. If the structure is used in this state, it is possible to tightly insert the lure 3120 of the syringe body 3010 into the packing 3052 of the direct connection-type port 3500 while maintaining a visual check on the syringe body 3010 and the lure 3120, since the lock connector 3020 can be kept on the syringe body 3010 and prevented from interfering.

Further, if the configuration of the lock connector-attached syringe 3001 shown in FIG. 22 is used, attachment to the needle hub 3060 is also simple in comparison to the conventional connector attached syringe because it is easy to visually check the lure 3120.
Note that the elastomer ring 3035 may be fixed to the external surface 3011 of the syringe body 3010 rather than the lock connector 3020. In this case, the internal surface 3200B of the lock connector makes contact with and slides over the elastomer ring 3035.

### Seventh Embodiment

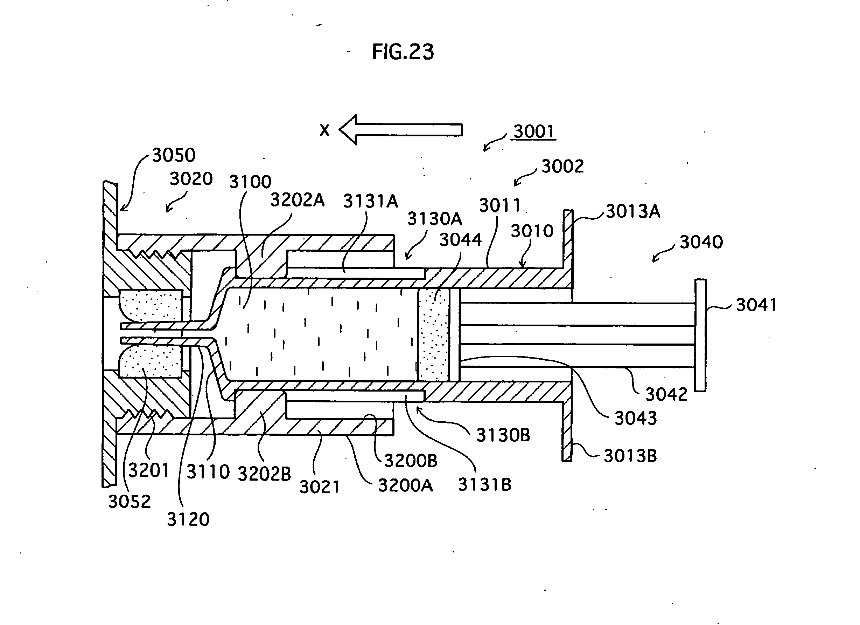

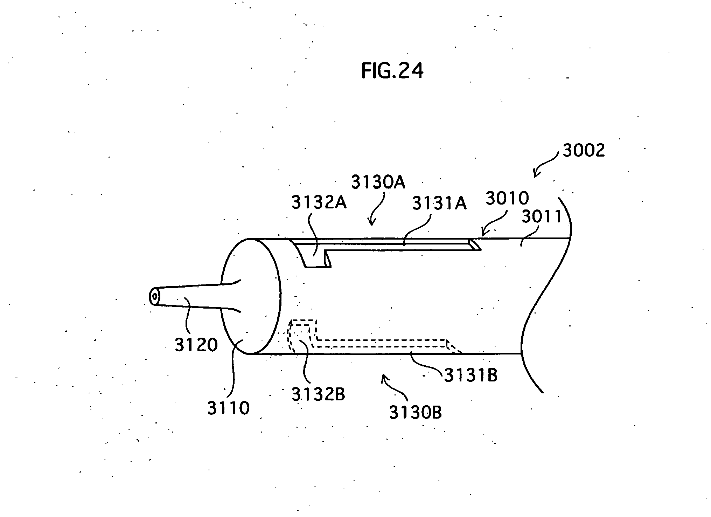

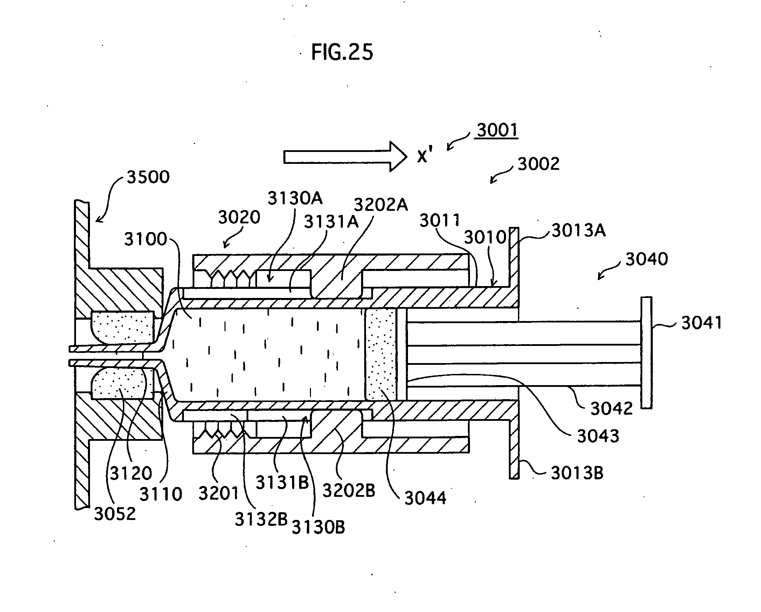

FIG. 23 shows the construction of a lock connector-attached syringe connected to the fixed connection-type port 3050 of a transfusion line system. FIG. 24 shows an external view of the syringe body. Further, FIG. 25 shows the construction of the lock connector-attached syringe connected to the direct connection-type port 3500 of a transfusion line system. In the seventh embodiment, a pair of guiding grooves 3130A and 3130B are symmetrically formed relative to the axial direction of the syringe in the external surface 3011 of the syringe body 3010, as shown in FIG. 23. A rib-shaped protrusion 3202A and a rib-shaped protrusion 3202B are formed on the internal surface 3200B of the lock connector 3020 so as to fit into the guiding groove 3130A and guiding groove 3130B. Otherwise, the construction is similar to that of the fifth embodiment.

The rib-shaped protrusion 3202A and rib-shaped protrusion 3 202B are both rectangular parallelepiped in shape, have a main surface extending in the axial direction of the syringe, and are formed integrally with the connector body 3021 by injection molding. The protrusion 3202A and protrusion 3202B are designed have a size and thickness that allows edge parts of the protrusion 3202A and protrusion 3202B to be fitted in the guiding grooves with a moderate frictional force. Note that the protrusions are not limited to this shape described here, and can take any form that enables engagement with the guiding groove 3130A and guiding groove 3130B.

The guiding groove 3130A and guiding groove 3130B, on the other hand, are provided by processing the external surface 3011 of the syringe body 3010 to form indented sections, as shown in FIG. 24. An exemplary pattern for the guiding groove 3130A and guiding groove 3202B is one in which a band- shaped return groove 3131A and a band-shaped return groove 3131B that extend in the syringe axial direction, and an engagement groove 3132A and an engagement groove 3132B that extend in the syringe circumferential direction are provided, the engagement groove 3132A and engagement groove 3132B being formed at the tip end of the syringe so as to communicate with the return groove 3131A and return groove 3131B, respectively. The width of the return groove 3131A and return groove 3131B is slightly narrower than the thickness of the protrusion 3202A and protrusion 3202B so that combining the respective grooves and protrusions gives rise to at least a certain frictional force. Further, the width and size of the engagement groove 3132A and engagement groove 3132B are set to be slightly smaller than the protrusion 3202A and protrusion 3202B such that the engagement groove 3132A and engagement groove 3132B and the protrusion 3202A and protrusion 3202B can be engaged with an moderate clicking.

Note that in the exemplary construction of FIG. 24, the engagement groove 3132A and engagement groove 3132B can be engaged with the protrusions 3202A and 3202B by rotating the connector body 3021 in a clockwise direction looking from the tip of the syringe. Also, the return grooves 3131A and 3131B are not limited to having a linear form, and may for instance describe a curve.
According to the seventh embodiment having the above construction, the following effects are obtained by sliding the syringe body 3010 and the connector body 3021 relative to each other with the protrusion 3202A and protrusion 3202B fitted into the guiding groove 3130A and guiding groove 3130B, respectively.

Firstly, if the operator grips the connector body 3021 while holding the syringe body 3010 and slides the connector body 3021 in the X direction toward the tip of the syringe, the thread part 3201, which is formed in the internal surface 3200B of the connector body 3021, can be slid to a position corresponding to the lure 3120 of the syringe body 3010. Then, by using the structure in this state and screwing the thread part 3201 of the connector body 3021 onto the fixed connection-type port 3050, the operator can tightly insert the lure 3120 of the syringe body 3010 into the packing 3052 of the fixed connection-type port 3050, while having the syringe body 3010 held by the frictional forces generated between the return groove 3131A and the protrusion 3202A and the return groove 3131B and the protrusion 3202B. Moreover, if the operator rotates the lock connector 3020 so as to twist the lock connector 3020 around the external surface of the syringe body 3010 either in a clockwise or anticlockwise direction (clockwise in the example of FIG. 24), the protrusion 3202A and protrusion 3202B fit into the engagement groove 3132A and engagement groove 3132B, and the lock connector 3020 and the syringe body 3010 lock with a moderate click and are fixed together without any play.

In the seventh embodiment, the operator can pre-connect the pre-filled syringe 3002 to the fixed connection-type port 3050 with a high level of stability using the operations necessitated by this type of locking mechanism, and press the plunger 3040 an appropriate amount into the syringe body 3010 to administer a required amount of the medication 3100 into the fixed connection-type port 3050 with a high level of stability.
Secondly, in the second embodiment the following effects can be obtained when the operator performs the above operations in the opposite order.

Specifically, if the operator rotates the lock connector 3020, twisting it on the surface of the syringe body 3010 in either a clockwise or anticlockwise direction (anticlockwise in the example of FIG. 24), the protrusion 3202A and protrusion 3202B detach from the engagement groove 3132A and engagement groove 3132B. If the operator slides the connector body 3021 in the X' direction to slide the protrusion 3202A and protrusion 3202B in the guiding groove 3131A and guiding groove 3131B towards the posterior end of the syringe, the lure 3120 can be exposed to the exterior, and consequently, the operator can easily check the position of the lure 3120 when connecting the syringe to the direct connection-type port 3500. Moreover, bes ides the direct connection-type port 3500, the syringe can similarly be attached to the needle hub 3060.

Note that in the seventh embodiment a construction was disclosed in which the lock connector 3020 was caused to slide with over the syringe body 3010 by forming the protrusion 3202A and protrusion 3202B in the lock connector 3020 and the guiding groove 3130A and guiding groove 3130B in the syringe body 3010. However, in the present invention similar effects are achieved even when the lock connector and syringe are joined by conversely forming the guiding grooves, each having a return groove and an engagement groove, in an internal surface of the connector, and the protrusions on the exterior surface of the syringe body.

### Eighth Embodiment

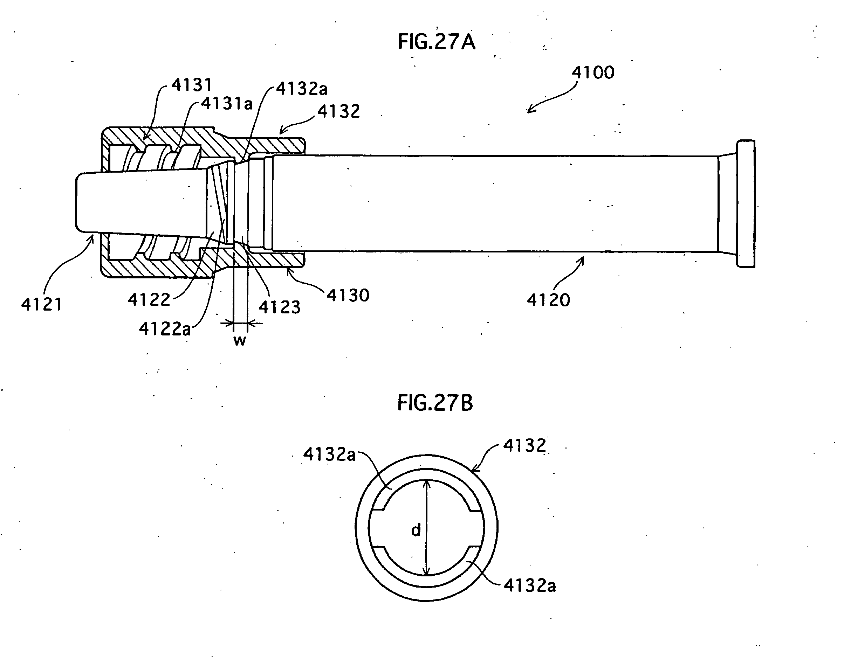

FIGs. 27A and 27B show the construction of a syringe 4100 of the eighth embodiment. This syringe 4100 is constituted from a syringe part 4120 and a lock part 4130 attachable at the tip end of the syringe part 4120. The syringe 4100 can be fixedly connected to a female taper connector (not depicted in the drawings) using the lock part 4130.

The syringe part 4120 is tubular and has a plunger (not depicted in the drawings) inserted from a posterior end of the syringe part 4120.
Further, a tapered lure 4121 for insertion into the female taper connector is formed at the tip end of the syringe part 4120, and a wedge part 4122 and a neck part 4123 to for engaging with the lock part 4130 are annularly formed in an external wall in a base section of the tapered lure 4121.

On the other hand, the lock part 4130 is annular and surrounds the tip section of the syringe part 4120. At a tip end section 4131 of the lock part 4130, a thread groove 4131a is formed on an internal surface to enable screwing onto the female thread of the tapered connector.
Further, a posterior end section 4132 of the lock part 4130 has an internal diameter which is similar to the external diameter of the tip section of the syringe part 4120, and has an enaging protrusion 4132a that fits into the neck part 4123 and engages the wedge part 4122. The engaging protrusion 4132a is annularly formed around the internal surface of the posterior end section 4132 and has cut-away sections, as shown in FIG. 27B. The internal diameter d is set to be slightly smaller than an external diameter of the wedge part 4122.

According to this construction, when attaching the lock part 4130 to the syringe part 4120, the engaging protrusion 4132a is passed over the wedge part 4122 and fitted into the neck part 4123 to engage the wedge part 4122, by pushing the lock part 4130 in a syringe axial direction.
Note that it is preferable to form a spiral groove 4122a in an exterior sloping surface of the wedge part 4122, as shown in FIG. 27A. This makes it easier to pass the lock part 4130 over the over the wedge part 4122 by rotating the lock part 4130 with the engaging protrusion 4132a aligned with the spiral groove 4122a. In this case, it is preferable to only form the spiral groove 4122a to part way down the tip end sloping surface of the wedge part 4122 and to not form a spiral groove in the posterior end, so that the engaging protrusion 4132a once fitted to the neck part 4123 is not easily removed.

The slope of the spiral groove 4122a in the longitudinal direction preferably faces in the opposite direction to the slope of the thread groove 4131a of the lock part 4130. This makes it more difficult to detach the lock part 4130 after attachment, when fixing the syringe 4100 to a connection target.
The width W (length in the axial direction) of the neck part 4123 is set to be larger than the width of the protrusion 4132a. This enables the lock part 4130 attached to the syringe part 4120 to be rotated freely with the engaging protrusion 4132a engaged with the wedge part 4122.

According to this construction, when the syringe 4100 is to be attached to or detached from the female tapered connector, the operator is able to attach or detach the syringe 4100 by rotating only the lock part 4130 to screw or unscrew the lock part 4130 in onto or from the female connector.
Hence, the attachment and detachment operations can be performed easily and can also be performed one handed. Moreover, when removing the syringe 4100 from the female tapered connector, the operator can perform the removal without touching the female tapered connector.
Consequently, the syringe 4100 has the effect of preventing the operator from mistakenly pricking their finger and coming into contact with infectious material.
Further, with the lock part 4130 screwed onto the female tapered connector, the syringe part 4120 is securely fixed to the female tapered connector by the lock part 4130.
Note that it is of course possible to use the syringe part 4120 on the lure slip coinfusion port 1210 or the like with the lock part 4130 removed from the syringe 4120 part.
Special embodiments:
1. A medical syringe comprising:
   a syringe unit including a lure and a syringe body; and a cylindrical connection supporting member that is slidably or detachably provided on the syringe unit, and increases a holding force of the lure when the syringe unit is in a connected state.
2. The medical syringe of embodiment 1, wherein in the slidable construction, the connection supporting member is formed to be slid along the lure or the syringe body in a state of being movable between a first position near a tip of the lure and a second position away from the tip of the lure, while remaining engaged with the syringe unit.
3. The medical syringe body of embodiment 1, wherein in the detachable construction, the connection supporting member is formed to be detached from the syringe unit using a predetermined disengagement operation.
4. A medical syringe comprising:
   a syringe unit including a lure and a syringe body; and a cylindrical connection supporting member that is rotatably provided on the syringe unit, and increases the holding force of the lure when the syringe unit is in a connected state.
5. The medical syringe of embodiment 4, wherein is slidably or detachably provided on the syringe unit.
6. A medical syringe comprising: a syringe unit including a lure for inserting into a connection target, and a syringe body; and a connection supporting member that increases a holding force of the lure when the syringe unit is connected to the connection target, wherein the connection supporting member is slidably provided on the lure or the syringe body in a state of being movable between a first position near a tip of the lure and a second position away from the tip of the lure, and when the connection supporting member is moved to the second position, the tip of the lure is exposed.
7. The medical syringe of embodiment 6, further comprising: a contact part that is provided on the lure or on the syringe body, and contacts the connection supporting member when the connection supporting member is moved to the first position, to prevent movement of the connection supporting member from the first position towards the tip of the lure.
8. The medical syringe of embodiment 7, wherein the sliding of the connection supporting member is realized by inserting the lure or syringe body into a through hole provided in the connection member, the contact part is a large diameter part provided on an external of the lure or the syringe body, and spiral grooves are provided in a peripheral surface of the contact part.
9. The medical syringe of embodiment 6, wherein when in the second position, the connection supporting member is fitted to the lure or the syringe body by a holding force that is at least sufficient to prevent the connection supporting member dropping under own weight.
10. A medical syringe comprising: a syringe unit including a lure for inserting into a connection target, and a syringe body; and a connection supporting member that increases a holding force of the lure when the syringe unit is connected to the connection target wherein one of the lure and the connection supporting member has a small diameter portion sandwiched between large diameter portions, the other of the lure and the connection supporting member has a first member that has a first opening in a main surface thereof, and a second member that has a second opening in a main surface thereof, the first and second members oppose one another with the main surfaces thereof near or contacting one another, and at least one of the members is displaceable relative to the other member, such that a hole in plan view formed by the first and second openings overlapping changes between a first size that permits insertion of the large diameter portion and a second size that prevents insertion of the large diameter portion.
11. The medical syringe of embodiment 10, wherein the first and second members are included in the connection supporting member, the large and small diameter portions are included in the lure, the second member is a cylindrical member with a base, and includes a locking mechanism for engaging the connection target, the second opening is in the base :of the second member, the first member has an elastic body that applies a force, such that the hole is the second size.
12. The medical syringe of embodiment 10, wherein the first and second members are included in the connection supporting member, the large and small diameter portions are included in the lure, the connection supporting member has a third member that includes a locking mechanism for engaging the connection target, the first and second members are slidably provided in the third member, and have respective elastic bodies that apply forces, such that the hole is the second size.
13. A medical syringe comprising a lock connector for connecting the syringe to a connection target, wherein the lock connector includes a cylindrical connector body, a syringe body is inserted through the connector body and an elastic body is interposed between an external surface of the syringe body and an internal surface of the connector body, and the syringe body is held with the connector body by a restoring force of the elastic body.
14. The medical syringe of embodiment 13, wherein the elastic body is composed of a spring body.
15. The medical syringe of embodiment 13, wherein the elastic body is composed of an elastomer.
16. A medical syringe including a lock connector for connecting the syringe to a connection target, wherein the lock connector has a cylindrical connector body and a protrusion formed on an internal surface of the connector body, a syringe body has a return groove that extends in a syringe axial direction provided in an external surface thereof, and the lock connector is held so as to be moveable back and forth, the syringe body being inserted into the connector body, and the protrusion of the connector body being fitted in the return groove of the syringe body.
17. A medical syringe including a lock connector for connecting the syringe to a connection target, wherein the lock connector has a cylindrical connector body, and a return groove extending in a syringe axial direction is provided in an internal surface of the connector body, and a syringe body has a protrusion formed on an external surface thereof, and the lock connector is held so as to be moveable back and forth, the syringe body being inserted into the connector body/ and of in of the connector body.
18. The medical syringe of embodiment 16 or 17, wherein an engagement groove is provided that communicates with the return groove and extends in a syringe circumferential direction, and the syringe body and the connector body are engaged by the protrusion being fitted into the engagement groove.

## Claims

1. A medical syringe (1100) comprising:
a syringe unit including a lure (1140) for inserting into a connection target (1200) and, a syringe body; and
a connection supporting member (1130) that increases a holding force of the lure (1140) when the syringe unit is connected to the connection target (1200), wherein
the connection supporting member (1130) is slidably provided on the lure (1140) or the syringe body in a state of being movable between a first position near a tip of the lure (1140) and a second position away from the tip of the lure (1140),
when the connection supporting member (1130) is moved to the second position, the tip of the lure (1140) is exposed,
the connection supporting member (1130) has a through hole provided therein, and
the sliding of the connection supporting member (1130) is realized by inserting the lure (1140) or syringe body into the through hole, and further comprising:
a contact part provided on the lure (1140) or on the syringe body, the contact part having a larger diameter than a diameter of the through hole and contacting the connection supporting member (1130) when the connection supporting member (1130) is moved to the first position, to prevent movement of the connection supporting member (1130) from the first position towards the tip of the lure,
**characterized in that** a slit extending from the through hole is provided in the connection supporting member (1130).

2. The medical syringe (1100) of claim 1, wherein spiral grooves are provided in a peripheral surface of the contact part (1143).

3. The medical syringe (1100) of claim 2, wherein
when in the second position, the connection supporting member (1130) is fitted to the lure (1140) or the syringe body by a holding force that is at least sufficient to prevent the connection supporting member (1130) dropping under own weight.
